# EUROPÄISCHE PATENTANMELDUNG

(11) **EP 4 603 110 A2**
(43) Veröffentlichungstag der Anmeldung: **20.08.2025**
(21) Anmeldenummer: 25177968.2
(22) Anmeldetag: 05.10.2023
(51) Int. Cl.: A61L 2/26

(54) **FUNKTIONSELEMENT, BETA-BEHÄLTER, TRANSFERSYSTEM UND BARRIERESYSTEM**

(30) Priorität: 07.10.2022 DE 102022125968
(62) Teilanmeldung aus: 23786529.0
(71) Anmelder: GRONINGER & CO. GMBH, 74564 Crailsheim (DE)
(72) Erfinder: Borkmann, Denis, 74589 Satteldorf (DE); Merz, Armin, 73479 Ellwangen (DE)
(74) Vertreter: Witte, Weller & Partner Patentanwälte mbB

(57) **Zusammenfassung**

Die vorliegende Erfindung betrifft ein Funktionselement (64, 164) für einen Beta-Behälter (38, 138) eines Transfersystems (24, 124), wobei das Funktionselement (64, 164) an und/oder in einer Beta-Portöffnung (48) eines Beta-Ports (44) des Beta-Behälters (38, 138) anordenbar ist, wobei das Funktionselement (64, 164) eine Funktionselementöffnung (68) aufweist, wobei die Funktionselementöffnung (68) kleiner als die Beta-Portöffnung (48) ist. Des Weiteren betrifft die vorliegende Erfindung ein Funktionselement (264) für einen Beta-Behälter (238) eines Transfersystems (224), wobei das Funktionselement (264) an und/oder in einer Beta-Portöffnung (48) eines Beta-Ports (44) des Beta-Behälters (238) anordenbar ist, wobei das Funktionselement (264) einen oder mehrere Nährbodenträgerhalter (268) aufweist, wobei jeder Nährbodenträgerhalter (268) dazu eingerichtet ist, einen Nährbodenträger zu halten. Des Weiteren betrifft die vorliegende Erfindung einen Beta-Behälter (38, 138, 238) für ein Transfersystem (24, 124, 224). Des Weiteren betrifft die vorliegende Erfindung ein Transfersystem (24, 124, 224). Des Weiteren betrifft die vorliegende Erfindung ein Barrieresystem, insbesondere ein Isolatorsystem.

## Beschreibung

Die vorliegende Erfindung betrifft ein Funktionselement für einen Beta-Behälter eines Transfersystems. Des Weiteren betrifft die vorliegende Erfindung einen Beta-Behälter eines Transfersystems mit einem derartigen Funktionselement. Des Weiteren betrifft die vorliegende Erfindung ein Transfersystem mit einem derartigen Beta-Behälter. Des Weiteren betrifft die vorliegende Erfindung ein Barrieresystem, insbesondere ein Isolatorsystem, mit einem derartigen Transfersystem.

Unter einem Barrieresystem ist ein System zu verstehen, das eine physikalische und aerodynamische Barriere, z.B. mittels Luftüberdruck, zwischen einer externen Umgebung, beispielsweise einer externen Reinraumumgebung, und einem Arbeitsprozess bietet. Im Stand der Technik sind verschiedene Barrieresysteme bekannt. Ein Barrieresystem kann beispielsweise ein Isolator oder eine Barriere mit eingeschränktem Zugang, ein sogenanntes RABS, "Restricted Area Barrier System", sein. Das RABS kann ein offenes RABS oder ein geschlossenes RABS sein.

Die vorliegende Erfindung beschäftigt sich in erster Linie mit aseptischen Isolatoren als Barrieresysteme. Die vorliegende Erfindung kann aber auch Anwendung in anderen Barrieresystemen finden.

Unter dem Begriff "Isolator" ist allgemein ein Behälter zu verstehen, der gegenüber dem umgebenden Arbeitsraum hermetisch und gasdicht abgeschlossen ist. Innerhalb eines Isolators kann eine definierte Atmosphäre zur Bearbeitung empfindlicher oder gefährlicher Produkte erzeugt werden.

In diesem Kontext werden Isolatoren üblicherweise in der biopharmazeutischen Prozesstechnik, beispielsweise als Teil einer Füllanlage mit mehreren Prozess- und Verarbeitungsstationen, verwendet, um eine hochreine oder sterile, sprich keimfreie Umgebung zu schaffen.

In derartigen Füllanlagen können beispielsweise Behälter, insbesondere Vials, Karpulen, Fläschchen, Spritzen und/oder dergleichen, mit einem Produkt, vorzugsweise einem pharmazeutischen oder kosmetischen Produkt, insbesondere einer Flüssigkeit oder einem Pulver, befüllt und dann mit einem Verschlusselement, beispielsweise einem Stopfen oder einer Verschlusskappe, insbesondere einer Bördelkappe, verschlossen werden.

Pharmazeutische Füllanlagen stehen in der Regel in einer keimarmen Umgebung. Insbesondere muss im Füllbereich innerhalb des Isolators eine keimfreie Umgebung vorliegen. Dieser Zustand wird überwacht, indem an den kritischen Stellen Nährbodenträger, auch Keimsammler genannt, platziert werden. Nährbodenträger können beispielsweise Petrischalen sein, die einen Nährboden aufweisen. Wenn ein Keim auf den Nährboden kommt, findet beim anschließenden Ausbrüten ein Wachstum des Keims statt, wodurch eine Kontamination rückwirkend nachgewiesen werden kann. Ein derartiges Überwachen einer keimfreien Umgebung wird als "Keimmoniotoring" oder "mikrobiologisches Monitoring" bezeichnet.

Zum Transfer von Objekten, wie beispielsweise Verschlusselementen oder Nährbodenträger, in den Isolator oder aus dem Isolator heraus können Transfersysteme verwendet werden. Ein Transfersystem kann eine Transferschleuse aufweisen, die von außen an den Isolator koppelbar ist. Über die Transferschleuse können Objekten in den Isolator hinein oder aus dem Isolator heraus transferiert werden. Ein Transfersystem kann beispielsweise als ein Portsystem, insbesondere als ein Alpha-Beta-Portsystem ausgestaltet sein. Derartige Transfersysteme weisen einen Alpha-Port und einen Beta-Behälter auf. Der Beta-Behälter dient als Transferschleuse. Der Beta-Behälter weist einen Beta-Port auf. Der Alpha-Port ist vorzugsweise an einer Isolatorwand des Isolators angeordnet, die einen Innenraum des Isolators umgibt. Der Alpha-Port und der Beta-Port sind miteinander koppelbar, um den Innenraum des Isolators mit einem Innenvolumen des Beta-Behälters zu verbinden. Im gekoppelten Zustand können Objekte aus dem Beta-Behälter in den Isolator eingebracht werden oder Objekte aus dem Isolator in den Beta-Behälter herausgebracht werden.

Zum Zuführen von Verschlusselementen, die in einem Beta-Behälter bereitgestellt werden, können Zuführeinrichtungen verwendet werden. Eine Zuführeinrichtung kann innerhalb des Isolators angeordnet sein und von Innen an dem Alpha-Port angeordnet werden. Die Zuführeinrichtung kann beispielsweise eine Rutsche oder ein Rohr aufweisen. Die Verschlusselemente können dann aus dem Beta-Behälter über die Zuführeinrichtungen in den Isolator eingebracht bzw. zugeführt werden.

Derartige Zuführeinrichtungen sind im Stand der Technik bekannt.

Beispielsweise zeigt die Druckschrift DE 10 2021 101 384 B3 ein System zum Transport von sterilen schüttfähigen Verschlusselementen aus einer Umgebung eines Isolators in einen Innenraum des Isolators, umfassend einen Behälter zur Speicherung einer Vorrats-Menge von Verschlusselementen in der Umgebung des Isolators, eine Isolatoröffnung und eine Sammeleinrichtung zum Sammeln der Verschlusselemente und zur Bereitstellung der Verschlusselemente in dem Innenraum des Isolators, mit einer Dosiervorrichtung zur Steuerung einer aus dem Behälter durch die Isolatoröffnung hindurch und in die Sammeleinrichtung zu transportierenden Soll-Menge von Verschlusselementen.

Des Weiteren zeigt die Druckschrift EP 3 581 339 B1 ein Transfersystem für dichtes Gehäuse, wobei das dichte Gehäuse ein erstes geschlossenes Volumen definiert und wenigstens eine dichte Verbindungsvorrichtung aufweist, die dazu bestimmt ist, das erste geschlossene Volumen mit einem zweiten geschlossenen Volumen zu verbinden, wobei das Transfersystem dazu bestimmt ist, in dem Gehäuse angeordnet zu werden, wobei das Transfersystem mindestens einen Arm aufweist, der dazu bestimmt ist, über ein erstes Drehgelenk, das eine erste Drehachse aufweist, drehbar an einer Wand des dichten Gehäuses gelagert zu werden, wobei das Transfersystem eine Rutsche aufweist, wobei die Rutsche einen Andockrand und einen Schüttrand aufweist, wobei der Andockrand dafür ausgelegt ist, mit der abgedichteten Verbindungsvorrichtung zusammenzuwirken, wobei das Transfersystem ein zweites Drehgelenk zwischen dem Arm und der Rutsche aufweist, wobei das zweite Drehgelenk eine zweite Drehachse aufweist.

Zum Zuführen oder Ausführen von Nährbodenträgern in bzw. aus einem Isolator kann ebenfalls ein Portsystem verwendet werden. Die Nährbodenträger können insbesondere in einem Beta-Behälter bereitgestellt werden. Im Isolator kann beispielsweise eine Handhabungseinrichtung vorgesehen sein, die die Nährbodenträger aus dem Beta-Behälter entnehmen oder in diesen zurücksetzen kann. Alternativ können Nährbodenträgern auch manuell mittels Handschuheingriff aus dem Beta-Behälter entnommen oder in diesen zurücksetzen werden.

Beispielsweise zeigt die Druckschrift DE 10 2020 102 758 B4 ein Verfahren zum automatisierten Keimmonitoring in einem Isolator, wobei der Isolator eine Transferschleuse aufweist, wobei das Verfahren die folgenden Schritte aufweist: erstes Bereitstellen mindestens eines Nährbodenträgerhalters an jeweils einer ersten Position innerhalb des Isolators; zweites Bereitstellen mindestens eines Nährbodenträgers innerhalb der Transferschleuse; erstes robotergestütztes Transferieren jeweils eines einzelnen Nährbodenträgers des mindestens einen Nährbodenträgers von der Transferschleuse zu einem freien Nährbodenträgerhalter des mindestens einen Nährbodenträgerhalters; und erstes robotergestütztes Anordnen des transferierten Nährbodenträgers in dem freien Nährbodenträgerhalter. Des Weiteren zeigt diese Druckschrift ein System zum automatisierten Keimmonitoring in einem Isolator und ein Computerprogramm.

Die bekannten Transfersysteme lassen aber noch Raum für Verbesserungen, insbesondere hinsichtlich des strukturellen Aufbaus, des Transfers von Objekten und der Betriebssicherheit.

Vor diesem Hintergrund ist es eine Aufgabe der vorliegenden Erfindung den strukturellen Aufbau eines Transfersystems und eines Barrieresystems zu verbessern. Des Weiteren ist es eine Aufgabe der vorliegenden Erfindung den Transfer von Objekten in einem Transfersystems und einem Barrieresystem zu verbessern. Des Weiteren ist es eine Aufgabe der vorliegenden Erfindung die Betriebssicherheit eines Transfersystems und eines Barrieresystems zu verbessern.

Gemäß einem ersten Aspekt der vorliegenden Erfindung wird ein Funktionselement für einen Beta-Behälter eines Transfersystems bereitgestellt, wobei das Funktionselement an und/oder in einer Beta-Portöffnung eines Beta-Ports des Beta-Behälters anordenbar ist, wobei das Funktionselement eine Funktionselementöffnung aufweist, wobei die Funktionselementöffnung kleiner als die Beta-Portöffnung ist.

Gemäß einem zweiten Aspekt der vorliegenden Erfindung wird ein Funktionselement für einen Beta-Behälter eines Transfersystems bereitgestellt, wobei das Funktionselement an und/oder in einer Beta-Portöffnung eines Beta-Ports des Beta-Behälters anordenbar ist, wobei das Funktionselement einen oder mehrere Nährbodenträgerhalter aufweist, wobei jeder Nährbodenträgerhalter dazu eingerichtet ist, einen Nährbodenträger zu halten.

Gemäß einem dritten Aspekt wird ein Beta-Behälter für ein Transfersystem bereitgestellt, wobei der Beta-Behälter ein Innenvolumen, eine Außenwand, die das Innenvolumen umschließt, einen Beta-Port, der mit einem Alpha-Port des Transfersystems koppelbar ist, und das Funktionselement nach dem ersten oder zweiten Aspekt aufweist.

Gemäß einem vierten Aspekt wird ein Transfersystem bereitgestellt, wobei das Transfersystem einen Alpha-Port und den Beta-Behälter nach dem dritten Aspekt aufweist.

Gemäß einem fünften Aspekt wird ein Barrieresystem bereitgestellt, wobei das Barrieresystem das Transfersystem nach dem vierten Aspekt aufweist. Das Barrieresystem ist insbesondere ein Isolatorsystem mit einem Isolator.

Der Isolator kann einen Innenraum aufweisen. Der Isolator kann eine Isolatorwand aufweisen, die den Innenraum umschließt bzw. umgibt. Die Isolatorwand trennt den Innenraum von einer äußeren Umgebung, die den Isolator umgibt. Der Isolator ist vorzugsweise ein aseptischer Isolator. Ein aseptischer Isolator weist eine hochreine oder sterile, sprich keimfreie Umgebung in dem Innenraum auf.

Der Isolator kann vorzugsweise Teil einer Füllanlage mit mehreren Prozess- und Verarbeitungsstationen sein. Die Füllanlage kann beispielsweise eine Anlage zum Füllen und Verschließen von Behältern mit einer pharmazeutischen oder kosmetischen Substanz sein. Die Anlage kann insbesondere eine Füllstation und mindestens eine Verschließstation aufweisen.

Das Transfersystem dient zum Transfer von Objekten, insbesondere von Verschlusselementen oder Nährbodenträgern, in den Isolator oder aus einem Isolator heraus. Das Transfersystem weist dazu den Alpha-Port und den Beta-Behälter auf.

Der Alpha-Port kann an der Isolatorwand des Isolators angeordnet sein. Der Alpha-Port weist eine Alpha-Portöffnung auf. Die Alpha-Portöffnung bildet einen Durchgang durch die Isolatorwand. Mit anderen Worten ist der Isolator durch die Alpha-Portöffnung von außen zugänglich. Die Alpha-Portöffnung kann eine beliebige Form aufweisen. Beispielsweise kann die Alpha-Portöffnung kreisförmig, elliptisch oder rechteckig sein. In einer bevorzugten Ausführungsform ist die Alpha-Portöffnung kreisförmig ausgebildet.

Der Alpha-Port kann des Weiteren einen Alpha-Portgrundkörper aufweisen. Der Alpha-Portgrundkörper kann vorzugsweise ringförmig ausgebildet sein. Insbesondere kann sich der Alpha-Portgrundkörper durch die Isolatorwand hindurch erstrecken. Der Alpha-Portgrundkörper weist eine Innenseite und eine Außenseite auf. Die Innenseite ist dem Innenraum zugewandt. Die Außenseite ist der äußeren Umgebung zugewandt. Die Außenseite ist insbesondere eine dem Beta-Port zugewandte Seite des Alpha-Ports. Der Alpha-Portgrundkörper kann die Alpha-Portöffnung aufweisen. Insbesondere kann die Alpha-Portöffnung durch eine Aussparung in dem Alpha-Portgrundkörper gebildet sein, die sich von der Innenseite zu der Außenseite des Alpha-Portgrundkörpers erstreckt. Insbesondere umgibt der Alpha-Portgrundkörper die Alpha-Portöffnung. Der Alpha-Port kann an der Außenseite einen Alpha-Portflansch aufweisen. Insbesondere kann der Alpha-Portgrundkörper den Alpha-Portflansch auf der Außenseite ausbilden. Der Alpha-Portflansch umgibt die Alpha-Portöffnung.

Der Alpha-Port kann des Weiteren eine Tür aufweisen. Die Tür ist an der Alpha-Portöffnung des Alpha-Ports angeordnet. Die Tür dient zum Öffnen und Verschließen der Alpha-Portöffnung. Vorzugsweise ist die Tür bewegbar, insbesondere schwenkbar, an dem Alpha-Portgrundkörper des Alpha-Ports angeordnet. Die Tür kann bewegt werden, um die Alpha-Portöffnung zu Öffnen oder zu Verschießen. In einem geschlossenen Zustand verschließt die Tür die Alpha-Portöffnung dicht. Zum Bewegen der Tür kann der Alpha-Port beispielsweise eine Antriebseinrichtung aufweisen.

Der Beta-Behälter weist ein Innenvolumen auf. In dem Innenvolumen können beispielsweise Objekte, wie Verschlusselemente oder Nährbodenträger, angeordnet werden. Der Beta-Behälter kann eine Außenwand aufweisen. Die Außenwand umgibt bzw. umschließt das Innenvolumen. Insbesondere isoliert die Außenwand das Innenvolumen gegenüber einer äußeren Umgebung, die den Beta-Behälter umgibt. Die Außenwand kann beispielsweise flexibel ausgestaltet sein. Insbesondere kann die Außenwand ein flexibler Beutel sein. Der flexible Beutel kann aus Kunststoff bestehen. Der flexible Beutel kann beispielsweise ein Sterilbeutel sein. Alternativ kann die Außenwand starr ausgebildet sein. Insbesondere kann die Außenwand als ein starres Gehäuse ausgebildet sein. Das Gehäuse kann aus einem Kunststoff oder einem Metall, beispielsweise Aluminium oder Edelstahl, bestehen.

Der Beta-Behälter weist den Beta-Port auf. Der Beta-Port kann an der Außenwand des Beta-Behälters angeordnet sein. Der Beta-Port weist eine Beta-Portöffnung auf. Die Beta-Portöffnung bildet einen Durchgang durch die Außenwand. Mit anderen Worten ist das Innenvolumen durch die Beta-Portöffnung von außen zugänglich. Die Beta-Portöffnung kann eine beliebige Form aufweisen. Beispielsweise kann die Beta-Portöffnung kreisförmig, elliptisch oder rechteckig sein. In einer bevorzugten Ausführungsform ist die Beta-Portöffnung kreisförmig ausgebildet.

Der Beta-Port kann des Weiteren einen Beta-Portgrundkörper aufweisen. Der Beta-Portgrundkörper kann vorzugsweise ringförmig ausgebildet sein. Insbesondere kann sich der Beta-Portgrundkörper durch die Außenwand hindurch erstrecken. Der Beta-Portgrundkörper weist eine Innenseite und eine Außenseite auf. Die Innenseite ist dem Innenvolumen zugewandt. Die Außenseite ist der äußeren Umgebung zugewandt. Die Außenseite ist insbesondere eine dem Alpha-Port zugewandte Seite des Beta-Ports. Der Beta-Portgrundkörper kann die Beta-Portöffnung des Beta-Ports aufweisen. Insbesondere kann die Beta-Portöffnung durch eine Aussparung in dem Beta-Portgrundkörper gebildet sein, die sich von der Innenseite zu der Außenseite des Beta-Portgrundkörpers erstreckt. Insbesondere umgibt der Beta-Portgrundkörper die Beta-Portöffnung. Der Beta-Port kann an der Außenseite einen Beta-Portflansch aufweisen. Insbesondere kann der Beta-Portgrundkörper den Beta-Portflansch auf der Außenseite ausbilden. Der Flansch umgibt die Beta-Portöffnung.

Der Beta-Port kann des Weiteren ein Abdeckelement aufweisen. Das Abdeckelement ist an bzw. in der Beta-Portöffnung des Beta-Ports anordenbar. Das Abdeckelement dient zum Abdecken bzw. Verschließen der Beta-Portöffnung. Das Abdeckelement kann lösbar mit dem Beta-Portgrundkörper oder dem Beta-Portflansch koppelbar sein. Wenn das Abdeckelement mit dem Beta-Portgrundkörper oder dem Beta-Portflansch gekoppelt ist, verschließt das Abdeckelement die Beta-Portöffnung dicht und deckt diese vollständig ab.

Der Alpha-Port ist mit dem Beta-Port koppelbar. In einem gekoppelten Zustand ist das Innenvolumen des Beta-Behälters mit dem Innenraum des Isolators verbunden. In dem gekoppelten Zustand sind der Alpha-Port und der Beta-Port derart gekoppelt, dass das Innenvolumen und der Innenraum gegenüber der äußeren Umgebung isoliert sind. Insbesondere kann eine Dichtung zwischen dem Alpha-Port und dem Beta-Port angeordnet sein. Im gekoppelten Zustand kann der Beta-Portflansch an dem Alpha-Portflansch angeordnet sein. Insbesondere kann die Dichtung zwischen dem Alpha-Portflansch und dem Beta-Portflansch angeordnet sein.

Die Beta-Portöffnung und die Alpha-Portöffnung sind vorzugsweise gleich groß. In einer bevorzugten Ausführungsform sind die Beta-Portöffnung und die Alpha-Portöffnung kreisförmig ausgestaltet. Insbesondere können die Beta-Portöffnung und die Alpha-Portöffnung den gleichen Durchmesser aufweisen.

Zur Kopplung können der Alpha-Port und der Beta-Port zum Beispiel korrespondierende Kopplungselemente aufweisen, wobei ein oder mehrere Kopplungselemente des Alpha-Ports mit einem oder mehreren Kopplungselementen des Beta-Ports koppelbar sind. Die Kopplungselemente können an dem Alpha-Portgrundkörper und dem Beta-Portgrundkörper oder an dem Beta-Portflansch und dem Alpha-Portflansch angeordnet sein.

Die Tür und das Abdeckelement können auch koppelbar sein. Nur wenn die Tür und das Abdeckelement gekoppelt sind, lassen sich die Tür und das Abdeckelement gemeinsam bewegen. Insbesondere können diese derart bewegt werden, dass die Alpha-Portöffnung und die Beta-Portöffnung gemeinsam geöffnet und verschlossen werden können. Insbesondere können die Tür und das Abdeckelement miteinander koppeln, wenn der Alpha-Port und der Beta-Port miteinander gekoppelt werden. Dadurch können Alpha-Portöffnung und die Beta-Portöffnung gemeinsam geöffnet und verschlossen werden, wenn der Alpha-Port und der Beta-Port miteinander gekoppelt sind.

Zur Kopplung können die Tür und das Abdeckelement zum Beispiel korrespondierende Kopplungselemente aufweisen, wobei ein oder mehrere Kopplungselemente der Tür mit einem oder mehreren Kopplungselementen des Abdeckelements koppelbar sind. Die Kopplungselemente können beispielsweise einen Bajonettverschluss ausbilden.

In dem Beta-Behälter können Objekte, wie beispielsweise Verschlusselemente oder Nährbodenträger, bereitgestellt sein, die in den Isolator transferiert werden sollen. Die Objekte können dann im gekoppelten Zustand des Alpha-Ports und des Beta-Ports in den Isolator transferiert bzw. eingebracht werden. Des Weiteren können Objekte auch in dem gekoppelten Zustand aus dem Isolator in den Beta-Behälter transferiert bzw. eingebracht werden, um diese Objekte aus dem Isolator heraus zu transferieren.

Wenn im gekoppelten Zustand des Alpha- und Beta-Ports Objekte von dem Beta-Behälter in den Isolator transferiert werden oder von dem Isolator in den Beta-Behälter transferiert werden, werden die Objekte jeweils in einer Transferrichtung durch die Alpha-Portöffnung und die Beta-Portöffnung transferiert. Die Transferrichtung kann insbesondere parallel zu einer Richtung verlaufen, die sich von der Innenseite zu der Außenseite des Beta-Ports erstreckt.

Erfindungsgemäß ist für den Beta-Behälter ein zusätzliches Funktionselement bereitgestellt. Das Funktionselement ist vorzugsweise in dem Beta-Behälter angeordnet. Das Funktionselement dient dazu, den Transfer von Objekten zwischen dem Beta-Behälter und dem Isolator zu verbessern. Insbesondere dient das Funktionselement dazu, den Transfer aus dem Beta-Behälter in den Isolator und/oder den Transfer der Objekte aus dem Isolator in den Beta-Behälter zu verbessern.

Das Funktionselement ist an und/oder in der Beta-Portöffnung anordenbar. Vorzugsweise kann das Funktionselement zumindest teilweise in der Beta-Portöffnung angeordnet sein. Alternativ kann das Funktionselement auch in dem Innenvolumen des Beta-Behälters angrenzend an die Beta-Portöffnung angeordnet sein. Das Funktionselement kann an dem Beta-Portgrundkörper des Beta-Ports angeordnet sein. Insbesondere kann das Funktionselement an dem Beta-Portgrundkörper, vorzugsweise lösbar, befestigt sein. Alternativ kann der Beta-Portgrundkörper auch das Funktionselement ausbilden.

Das Funktionselement kann einen Funktionselementkörper aufweisen. Der Funktionselementkörper kann einen äußeren Rand aufweisen. Der äußere Rand kann an dem Beta-Portgrundkörper angeordnet sein. Insbesondere kann der äußere Rand umlaufend an dem Beta-Portgrundkörper in der Beta-Portöffnung anliegen, wenn das Funktionselement zumindest teilweise in der Beta-Portöffnung angeordnet ist.

Das Funktionselement nach dem ersten Aspekt weist zudem die Funktionselementöffnung auf. Bezüglich der Funktionselementöffnung können eine radiale Richtung, eine axiale Richtung und eine tangentiale Richtung, auch Umlaufrichtung genannt, definiert werden. Die radiale Richtung ist eine Richtung die sich von der Öffnung, insbesondere einem Mittelpunkt der Funktionselementöffnung radial nach außen erstreckt. Die axiale Richtung ist eine Richtung, in der sich die Funktionselementöffnung durch das Funktionselement hindurch erstreckt. Die Umlaufrichtung ist eine Richtung, die um die Funktionselementöffnung herum verläuft. Die radiale Richtung, die axiale Richtung und die Umlaufrichtung verlaufen senkrecht zueinander. Das Funktionselement ist vorzugsweise in dem Beta-Behälter derart anordenbar, dass die axiale Richtung parallel zu der Transferrichtung der Objekte verläuft.

Die Funktionselementöffnung ist kleiner als die Beta-Portöffnung. Insbesondere ist ein Querschnittsgeometrie der Funktionselementöffnung senkrecht zu der axialen Richtung kleiner als eine Querschnittsgeometrie der Beta-Portöffnung senkrecht zu einer Richtung von der Außenseite zu der Innenseite. Insbesondere ist ein Durchmesser der Funktionselementöffnung kleiner als ein Durchmesser der Beta-Portöffnung.

Der Funktionselementkörper des Funktionselements nach dem ersten Aspekt kann sich von der Funktionselementöffnung in der radialen Richtung nach außen erstrecken. Die Funktionselementöffnung kann insbesondere als eine Aussparung in dem Funktionselementkörper ausgebildet sein. Das Funktionselement nach dem ersten Aspekt kann einen Funktionselementflansch aufweisen, der die Funktionselementöffnung umgibt. Insbesondere kann der Funktionselementkörper den Funktionselementflansch aufweisen bzw. ausbilden.

Das Funktionselement nach dem ersten Aspekt kann in der axialen Richtung ein erstes axiales Ende und ein zweites axiales Ende aufweisen, die auf entgegengesetzten Seiten des Funktionselements angeordnet sind. Der Funktionselementflansch kann an dem ersten axialen Ende angeordnet sein. Der Funktionselementflansch umgibt die Funktionselementöffnung an dem ersten axialen Ende. Das Funktionselement kann in dem Beta-Behälter derart angerordnet sein, dass das erste axialen Ende dem Innenvolumen abgewandt und dem Alpha-Port zugewandt ist. Entsprechend kann das Funktionselement in dem Beta-Behälter derart angerordnet sein, dass das zweite axialen Ende dem Innenvolumen zugewandt und dem Alpha-Port abgewandt ist.

Objekte, wie beispielsweise Verschlusselemente, können in dem Beta-Behälter, insbesondere in dessen Innenvolumen, bereitgestellt werden. Wenn der Beta-Port mit dem Alpha-Port gekoppelt ist, können diese Objekte dann in den Isolator eingebracht werden. Dazu müssen diese durch die Beta-Portöffnung und die Alpha-Port-Öffnung transferiert werden. Das Funktionselement ist derart angeordnet, dass die Objekte auf dem Transferweg aus dem Innenvolumen in den Isolator durch die Funktionselementöffnung transferiert werden. Das Funktionselement nach dem ersten Aspekt eignet sich besonders für Verschlusselemente als zu transferierende Objekte.

Bei einem Beta-Behälter ohne ein Funktionselement entspricht ein Austrittsquerschnitt dieser Objekte der Größe des Beta-Ports, insbesondere der Querschnittsgeometrie des Beta-Ports. Unter einem Austrittsquerschnitt ist die Querschnittsfläche zu verstehen, die den Objekten beim Passieren der Beta-Portöffnung senkrecht zu einer Transferrichtung zur Verfügung steht. Die Funktionselementöffnung nun dient dazu, diesen Austrittsquerschnitt zu verkleinern. Mit anderen Worten bedeutet dies, dass bei einem Beta-Behälter mit einem Funktionselement nach dem ersten Aspekt der Austrittsquerschnitt verkleinert ist. Insbesondere wird dadurch die Querschnittsfläche des Austrittsquerschnitts von einem Rand der Beta-Portöffnung beabstandet.

Bei dem Transfer von Objekten in einem Transfersystem zwischen einem Beta-Behälter und einem Isolator ist es unter anderem bekannt, dass ein Kontakt der zu transferierenden Objekte mit einem sogenannten "Ring of concern" vermieden werden soll. Der "Ring of concern" umfasst beispielsweise die Kontaktflächen zwischen Alpha-Port und Beta-Port, insbesondere eine umlaufende Linie der Dichtung zwischen Alpha-Port und Beta-Port. Es ist insbesondere wünschenswert einen Kontakt der Objekte mit der Beta-Portöffnung und der Alpha-Port-Öffnung weitestgehend oder vollständig zu vermeiden.

Beispielsweise wurde bisher im Stand der Technik bei der Verwendung von Sterilbeuteln als Beta-Behälter ein innenliegender Schlauch verwendet, der im gekoppelten Zustand des Alpha- und Beta-Ports aus dem Sterilbeutel herausgehohlt werden kann, um den "Ring of concern" zu überbrücken. Der Schlauch muss allerdings mittels Handschuheingriffs von innerhalb des Isolators aus dem Sterilbeutel herausgeholt werden. Dies bedeutet, dass eine zusätzliche manuelle Bedientätigkeit notwendig ist, die zur potenziellen Verschleppung von Keimen durch den Handschuh führen kann.

Durch das Bereitstellen des Funktionselements nach dem ersten Aspekt in dem Beta-Behälter wird der "Ring of concern" ebenfalls überbrückt. Wie zuvor erläutert, wird mittels der Funktionselementöffnung der Austrittsquerschnitt verkleinert und insbesondere von einem Rand der Beta-Portöffnung beabstandet. Dadurch kann ebenfalls ein Kontakt der Objekte mit der Beta-Portöffnung und der Alpha-Port-Öffnung, insbesondere mit dem "Ring of concern", weitestgehend oder vollständig vermieden werden. Das Funktionselement nach dem ersten Aspekt überbrückt somit den "Ring of Concern", ohne dass eine manuelle Bedientätigkeit notwendig ist.

Bei Beta-Behältern, die ein starres Gehäuse aus beispielsweise Edelstahl aufweisen, wurde der "Ring of concern" bisher im Stand der Technik durch eine bestimmte Anordnung des Alpha-Ports und der Zuführeinrichtung überbrückt. Als Zuführeinrichtung wurde dazu eine Rutsche oder ein Schwenkrohr innerhalb des Isolators am Alpha-Port vorgesehen, die, vorzugsweise automatisch, in die Alpha-Portöffnung eingeführt, insbesondere eingeschwenkt, werden kann, um dadurch den "Ring of concern" zu überbrücken. Allerdings war es hier bisher notwendig, den Alpha-Port schräg anzuordnen, damit das Rohr in Fallrichtung eingeschwenkt werden kann und die kreisrunden Öffnungen von Alpha-Port und Rohr konzentrisch angeordnet sind. Hierzu war es bisher insbesondere erforderlich an einer vertikalen Isolatorwand einen Erker vorzusehen, mittels dem der Port schräg zur Isolatorwand angeordnet werden kann. Durch den Erker werden die Herstellungskosten des Isolators erhöht. Zudem bildet der Erker bezüglich der Laminarflow-Strömung innerhalb des Isolators eine kritische Stelle für Wirbel-Erzeugung. Des Weiteren ist durch den Erker auch die Reinigung aufgrund zusätzlicher Ecken und Kanten erschwert.

Durch das Bereitstellen des Funktionselements nach dem ersten Aspekt in dem Beta-Behälter ist diese spezielle Anordnung des Alpha-Ports nicht mehr notwendig. Wenn eine Zuführeinrichtung, beispielsweise ein Rohr, schräg in Alpha-Port eingeführt wird, wird der Eintrittsquerschnitt der Zuführeinrichtung verkleinert und ist dadurch nicht mehr in die Alpha-Portöffnung angepasst. Bei einem zylindrischen Rohr als Zuführeinrichtung kann das schräge Einführen zum Beispiel zu einem elliptischen Eintrittsquerschnitt führen.

Dies führt bei Beta-Behältern ohne Funktionselement dazu, dass Objekte, die die Beta-Portöffnung und die Alpha-Portöffnung passieren, nicht zwangsläufig in der Zuführeinrichtung transferiert werden, sondern neben der Zuführeinrichtung die Öffnungen passieren. Dadurch können diese in Kontakt mit dem "Ring of concern" kommen oder fallen daneben auf die Maschine oder verklemmen sich.

Durch das Bereitstellen des Funktionselements nach dem ersten Aspekt in dem Beta-Behälter kann vermieden werden, dass die Objekte in den Öffnungen des Alpha- und Beta-Ports neben der Zuführeinrichtung vorbei bewegt werden können. Dies wird insbesondere dadurch erreicht, dass durch das Funktionselement der Austrittsquerschnitt aus der Beta-Portöffnung ebenfalls verkleinert ist. Insbesondere kann die Funktionselementöffnung derart ausgebildet sein, dass der Austrittsquerschnitt kleiner oder gleich als der Eintrittsquerschnitt ist.

Das Funktionselement nach dem zweiten Aspekt weist einen oder mehrere Nährbodenträgerhalter auf. Jeder Nährbodenträgerhalter kann dazu eingerichtet sein, einen Nährbodenträger zu halten. Insbesondere kann in jedem Nährbodenträgerhalter ein Nährbodenträger aufgenommen werden. Das Funktionselement nach dem zweiten Aspekt dient somit dazu, in dem Beta-Behälter einen oder mehrere Nährbodenträger bereitzustellen.

An dem Funktionselementkörper können die ein oder mehreren Nährbodenträgerhalter angeordnet sein. Jeder Nährbodenträgerhalter kann beispielsweise ein Aufnahmeelement oder eine Auflagefläche für jeweils einen Nährbodenträger aufweisen. Die Nährbodenträgerhalter sind vorzugsweise auf einer dem Alpha-Port zugewandten Seite des Funktionselements angeordnet.

Das Funktionselement nach dem zweiten Aspekt kann somit dazu verwendet werden, einen oder mehrere Nährbodenträger in den Isolator hinein oder aus dem Isolator heraus zu transferieren.

Das Isolatorsystem kann innerhalb des Isolators eine Handhabungseinrichtung aufweisen, die dazu eingerichtet ist, die Nährbodenträger zu handhaben. Die Handhabungseinrichtung kann insbesondere als Handhabungsroboter ausgestaltet sein. Die Handhabungseinrichtung kann beispielsweise einen mehrachsigen Arm und einen Endeffektor aufweisen, der an einem Ende des Arms angeordnet ist. Der Endeffektor kann ein Greifwerkzeug, vorzugsweise einen Greifer, aufweisen. Mittels des Greifwerkzeugs können Nährbodenträger gegriffen und transferiert werden.

Die Handhabungseinrichtung kann insbesondere dazu eingerichtet sein, einen Nährbodenträger, der in einem der Nährbodenträgerhalter des Funktionselements angeordnet ist, zu greifen und zu einer Position innerhalb des Isolators zu transferieren. Die Handhabungseinrichtung kann auch dazu eingerichtet sein, einen Nährbodenträger, der an einer Position innerhalb des Isolators angeordnet ist, zu greifen und zu einem der Nährbodenträgerhalter des Funktionselements zu transferieren. Eine derartige Handhabungseinrichtung zum Transfer von Nährbodenträgern ist beispielsweise in der Druckschrift DE 10 2020 102 758 B4 der Anmelderin beschrieben. Insbesondere kann mittels einem derartig ausgestaltetem Isolatorsystem das Keimmonitoring, wie in der Druckschrift DE 10 2020 102 758 B4 der Anmelderin beschrieben, durchgeführt werden.

Mittels des Funktionselements nach dem zweiten Aspekt können somit Nährbodenträger in dem Beta-Behälter einfach und sicher bereitgestellt bzw. angeordnet werden.

Die eingangs gestellte Aufgabe wird somit vollumfänglich gelöst.

In einer ersten Ausgestaltung der Aspekte kann die Funktionselementöffnung kreisförmig ausgebildet sein.

Vorzugsweise können die Beta-Portöffnung und insbesondere auch die Alpha-Portöffnung ebenfalls kreisförmig ausgebildet sein. Insbesondere ist der Durchmesser der Funktionselementöffnung kleiner als der Durchmesser der Beta-Portöffnung. Der Durchmesser der Funktionselementöffnung kann 20% bis 80%, vorzugsweise 35% bis 65%, insbesondere 50%, des Durchmessers der Beta-Portöffnung betragen.

In einer weiteren Ausgestaltung der Aspekte kann die Funktionselementöffnung konzentrisch zu der Beta-Portöffnung anordenbar sein.

Vorzugsweise haben die Funktionselementöffnung und die Beta-Portöffnung die gleiche Form, wobei die Beta-Portöffnung größer als die Funktionselementöffnung ist. Der Begriff "konzentrisch" ist hier so zu verstehen, dass die Funktionselementöffnung und die Beta-Portöffnung symmetrisch zu einer Mittelachse angeordnet sind.

In einer weiteren Ausgestaltung der Aspekte kann das Funktionselement ringförmig oder trichterförmig ausgebildet sein.

Bei einer Trichterform sind beide Seiten des Trichters offen. Die Funktionselementöffnung ist durch die innere Aussparung der Ringform oder Trichterform ausgebildet. Der äußere Rand des Rings kann umlaufend in der Beta-Portöffnung angeordnet sein. Auf diese Weise kann das Funktionselement geeignet in die Beta-Portöffnung eingepasst werden.

In einer weiteren Ausgestaltung der Aspekte kann das Funktionselement als ein Einsatzelement ausgebildet sein, das in den Beta-Behälter, insbesondere in die Beta-Portöffnung einsetzbar ist.

Auf diese Weise können insbesondere bestehende Beta-Behälter, die bisher kein Funktionselement aufweisen, geeignet nachgerüstet werden, indem man das Funktionselement in diese einsetzt. Wenn das Funktionselement in den Beta-Behälter eingesetzt ist, kann der Beta-Behälter insbesondere gemeinsam mit dem Funktionselement sterilisiert werden.

In einer weiteren Ausgestaltung der Aspekte kann das Funktionselement einen Funktionselementkörper aufweisen, wobei der Funktionselementkörper einen äußeren Rand aufweist, wobei der Funktionselementkörper die Funktionselementöffnung umgibt oder an dem Funktionselementkörper die ein oder mehreren Nährbodenträgerhalter angeordnet sind.

Wie zuvor bereits beschrieben, kann die Funktionselementöffnung als eine Aussparung in dem Funktionselementkörper ausgebildet sein, die sich durch den Funktionselementkörper hindurch erstreckt. Alternativ können an dem Funktionselementkörper die ein oder mehreren Nährbodenträgerhalter angeordnet sein. Der Funktionselementkörper kann insbesondere die ein oder mehreren Nährbodenträgerhalter tragen oder ausbilden.

In einer weiteren Ausgestaltung der Aspekte kann der Funktionselementkörper einen äußeren Rand aufweisen, wobei der äußere Rand elastisch, insbesondere lamellenartig, ausgebildet ist.

Insbesondere können an dem äußeren Rand umlaufend eine Mehrzahl von Lamellen angeordnet sein. Wenn an dem Beta-Portgrundkörper in der Beta-Portöffnung innere Vorsprünge zum Beispiel als Kopplungselemente für das Abdeckelement oder das Funktionselement vorgesehen sind, ermöglicht die elastische Ausgestaltung, dass das Funktionselement in die Beta-Portöffnung trotzdem eingesetzt werden kann. An den inneren Vorsprüngen kann der elastische, äußere Rand einfach nach innen nachgeben. Nachdem das Funktionselement die inneren Vorsprünge beim Einsetzen passiert hat, federt der äußere Rand automatisch wieder nach außen.

In einer weiteren Ausgestaltung der Aspekte kann der Funktionselementkörper einen äußeren Rand aufweisen, wobei der äußere Rand komplementär zu der Beta-Portöffnung ausgebildet ist.

Insbesondere kann der äußere Rand in der Beta-Portöffnung umlaufend an dem Beta-Portgrundkörper angeordnet sein bzw. an diesem umlaufend anliegen. Auf diese Weise ist das Funktionselement in die Beta-Portöffnung eingepasst. Objekte, die zwischen dem Isolator und dem Innenvolumen transferiert werden, müssen damit die Funktionselementöffnung passieren. Insbesondere kann der äußere Rand in der radialen Richtung radial Außen angeordnet sein. Der äußere Rand kann vorzugsweise an dem zweiten axialen Ende angeordnet sein.

In einer weiteren Ausgestaltung der Aspekte kann das Funktionselement ein oder mehrere Befestigungselemente aufweisen, mittels denen das Funktionselement an dem Beta-Port, insbesondere an einem Beta-Portgrundkörper des Beta-Ports, befestigbar ist.

Auf diese Weise kann das Funktionselement, insbesondere in einem eingesetzten Zustand, fest mit dem Beta-Port verbunden werden. Dadurch wird das Funktionselement relativ zu dem Beta-Port in einer definierten Lage angeordnet und gehalten. Dies erleichtert den Transfer von Objekten aus dem Beta-Behälter in den Isolator oder aus dem Isolator in den Beta-Behälter. Insbesondere ist das Funktionselement mittels den Befestigungselementen lösbar an dem Beta-Portgrundkörper befestigbar. Der Beta-Portgrundkörper kann korrespondierende Aufnahmeelemente aufweisen. Die Befestigungselemente können in Eingriff mit den Aufnahmeelementen gebracht werden, um das Funktionselement zu befestigen. Je ein Befestigungselement kann in Eingriff mit einem entsprechenden Aufnahmeelement gebracht werden. Die Befestigungselemente können insbesondere hakenförmig ausgebildet sein. Vorzugsweise sind die Befestigungselemente an dem äußeren Rand des Funktionselementkörpers angeordnet.

In einer weiteren Ausgestaltung der Aspekte kann der Beta-Port das Funktionselement aufweisen, insbesondere wobei ein Beta-Portgrundkörper des Beta-Ports und das Funktionselement einstückig ausgebildet sind.

Mit anderen Worten können der Beta-Portgrundkörper und das Funktionselement integral geformt sein. In dieser Ausgestaltung ist das Funktionselement somit in dem Beta-Behälter integriert. Ein Beta-Behälter, der ein derart integriertes Funktionselement aufweist, eignet sich insbesondere zur mehrfachen Verwendung.

In einer weiteren Ausgestaltung der Aspekte kann der Beta-Port ein Abdeckelement zum Abdecken der Beta-Portöffnung aufweisen, wobei das Funktionselement zwischen dem Abdeckelement und dem Innenvolumen angeordnet ist.

Mittels des Abdeckelements kann die Beta-Portöffnung verschlossen werden. Das Funktionselement kann vorzugsweise an dem oder angrenzend an das Abdeckelement angeordnet sein. Das Abdeckelement dient somit zum dichten Abdecken bzw. Verschließen der Beta-Portöffnung. Insbesondere kann der Funktionselementflansch angrenzend an dem Abdeckelement angeordnet sein. Dadurch ist die Funktionselementöffnung so nah wie möglich an einem äußeren Ende der Beta-Portöffnung angeordnet. Auf diese Weise ist sichergestellt, dass aus dem Beta-Behälter zu transferierende Objekte nicht mit dem "Ring of concern" in Kontakt geraten.

In einer weiteren Ausgestaltung der Aspekte kann der Beta-Port ein Dichtungselement zum Abdichten der Kopplung zwischen dem Alpha-Port und dem Beta-Port aufweisen.

Das Dichtungselement kann vorzugsweise auf der Außenseite des Beta-Portgrundkörpers angeordnet sein. Insbesondere kann das Dichtungselement an dem Beta-Portflansch angeordnet sein. Insbesondere kann der Beta-Portgrundkörper auf der Außenseite eine um die Beta-Portöffnung umlaufende Nut aufweisen, in die das Dichtungselement eingelegt ist. Das Dichtungselement kann beispielsweise eine Lippendichtung sein. Im gekoppelten Zustand kann das Dichtungselement zwischen dem Alpha-Portflansch und dem Beta-Portflansch angeordnet sein. Auf diese Weise wird das Innenvolumen des Beta-Behälters und der Innenraum des Isolators gegenüber einer äußerten Umgebung des Isolators abgedichtet.

In einer weiteren Ausgestaltung der Aspekte kann der Isolator einen Innenraum und eine Isolatorwand aufweisen, die den Innenraum umschließt, wobei der Alpha-Port an der Isolatorwand angeordnet ist, wobei der Beta-Port von einer äußeren Umgebung des Isolators an den Alpha-Port koppelbar ist.

Wenn der Alpha-Port und der Beta-Port miteinander gekoppelt sind, können Objekte zwischen dem Beta-Behälter und dem Isolator transferiert werden.

In einer weiteren Ausgestaltung der Aspekte kann der Alpha-Port an einer vertikalen Wandfläche der Isolatorwand angeordnet sein.

Die vertikale Wandfläche verläuft in dem Isolator parallel zu einer vertikalen Richtung. Wie zuvor bereits beschrieben, kann der Alpha-Port aufgrund des Funktionselements an einer vertikalen Wand angeordnet sein. Der Alpha-Port muss somit nicht an einem Erker angeordnet werden.

In einer weiteren Ausgestaltung der Aspekte kann das Barrieresystem des Weiteren eine Zuführeinrichtung in dem Innenraum des Isolators aufweisen, wobei die Zuführeinrichtung von Innen an dem Alpha-Port anordenbar ist.

Die Zuführeinrichtung dient zum Zuführen von Objekten, insbesondere von Verschlusselementen, in den Isolator. Wenn die Zuführeinrichtung an dem Alpha-Port angeordnet ist, können die Objekte aus dem Beta-Behälter durch den Alpha- und Beta-Port über die Zuführeinrichtung in den Isolator zugeführt werden. Die Zuführeinrichtung kann dazu ein Rohr oder eine Rutsche aufweisen. Die Zuführeinrichtung kann einen Arm aufweisen, der schwenkbar an der Isolatorwand, vorzugsweise über dem Alpha-Port, angeordnet ist.

In einer weiteren Ausgestaltung der Aspekte kann die Zuführeinrichtung ein Rohr aufweisen, wobei ein erstes, offenes Ende des Rohrs an dem Alpha-Port anordenbar ist, insbesondere wobei das Rohr zylindrisch ausgebildet ist.

Insbesondere kann sich das erste Ende des Rohrs in die Alpha-Portöffnung hinein erstrecken. Über das Rohr können dann die Objekte aus dem Beta-Behälter in den Isolator eingebracht werden. Das Rohr weist das erste, offene Ende und ein zweites, offenes Ende auf. Die Objekte gehen beim Transfer an dem ersten, offenen Ende in das Rohr hinein und kommen dann an dem zweiten, offenen Ende aus dem Rohr wieder heraus.

In einer weiteren Ausgestaltung der Aspekte kann das Rohr der Zuführeinrichtung an dem Alpha-Port derart anordenbar sein, dass es sich im gekoppelten Zustand durch eine Alpha-Portöffnung des Alpha-Ports hindurch bis zu der Beta-Portöffnung erstreckt, insbesondere wobei das erste Ende des Rohrs an einem Funktionselementflansch des Funktionselements anordenbar ist.

Dadurch gehen Objekte, die ausgehend von dem Innenvolumen des Beta-Behälters die Funktionselementöffnung passieren, direkt in das Rohr der Zuführeinrichtung. Dadurch wird sichergestellt, dass die Objekte nicht in Kontakt mit dem "Ring of concern" kommen.

In einer weiteren Ausgestaltung der Aspekte kann das Rohr der Zuführeinrichtung an dem Alpha-Port derart anordenbar sein, dass das Rohr schräg zu einer horizontalen Richtung verläuft, insbesondere wobei das erste Ende des Rohrs eine elliptische Form aufweist.

Insbesondere ist hierbei das zweite Ende des Rohrs in der vertikalen Richtung tiefer angeordnet als das erste Ende des Rohrs. Dadurch können zuzuführende Objekte mittels ihrer Schwerkraft durch das Rohr hindurchbewegt werden. Wenn der Alpha-Port an einer vertikalen Wandfläche angeordnet ist, ist die Alpha-Portöffnung horizontal ausgerichtet. Mit anderen Worten verläuft die Transferrichtung in diesem Fall parallel zu einer horizontalen Richtung. Damit das erste Ende des Rohrs trotzdem flächig an dem Funktionselementflansch anordenbar ist, kann das erste Ende des Rohrs entsprechend schräg abgeschnitten sein. Dadurch ist das erste Ende des Rohrs ellipsenförmig ausgebildet. Insbesondere ist der Durchmesser der Funktionselementöffnung gleich oder kleiner als der kleinste Durchmesser der Ellipsenform des ersten Endes des Rohrs.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine isometrische Ansicht einer ersten Ausführungsform eines Isolatorsystems;
- Fig. 2: eine Längsschnittansicht des Isolatorsystems aus Fig. 1;
- Fig. 3: eine isometrische Ansicht des Isolatorsystems aus Fig. 1 mit weggeschwenkter Zuführeinrichtung;
- Fig. 4: eine isometrische Ansicht eines Transfersystems des Isolatorsystems aus Fig. 1;
- Fig. 5: eine isometrische Ansicht einer Rückseite des Transfersystems aus Fig. 4;
- Fig. 6: eine Längsschnittansicht des Transfersystems aus Fig. 4;
- Fig. 7: eine isometrische Ansicht eines Alpha-Ports des Transfersystems aus Fig. 4;
- Fig. 8: eine isometrische Ansicht einer Rückseite des Alpha-Ports aus Fig. 7;
- Fig. 9: eine isometrische Ansicht eines Beta-Behälters des Transfersystems aus Fig. 4;
- Fig. 10: eine Längsschnittansicht des Beta-Behälters aus Fig. 9;
- Fig. 11: eine isometrische Ansicht des Beta-Behälters aus Fig. 9 ohne Abdeckelement;
- Fig: 12: eine isometrische Ansicht eines Funktionselements des Beta-Behälters aus Fig. 9;
- Fig. 13: eine isometrische Ansicht einer Rückseite des Funktionselements aus Fig. 12;
- Fig. 14: eine Längsschnittansicht des Funktionselements aus Fig. 12;
- Fig. 15: eine isometrische Ansicht einer zweiten Ausführungsform eines Isolatorsystems;
- Fig. 16: eine Längsschnittansicht des Isolatorsystems aus Fig. 15;
- Fig. 17: eine isometrische Ansicht eines Transfersystems des Isolatorsystems aus Fig. 15;
- Fig. 18: eine isometrische Ansicht eines Beta-Behälters des Transfersystems aus Fig. 17;
- Fig. 19: eine isometrische Ansicht einer Rückseite des Beta-Behälters aus Fig. 18;
- Fig. 20: eine Längsschnittansicht des Beta-Behälters aus Fig. 18;
- Fig. 21: eine isometrische Ansicht einer dritten Ausführungsform eines Isolatorsystems;
- Fig. 22: eine isometrische Ansicht eines Transfersystems des Isolatorsystems aus Fig. 21;
- Fig. 23: eine isometrische Ansicht einer Rückseite des Transfersystems aus Fig. 22;
- Fig. 24: eine isometrische Ansicht eines Beta-Behälters des Transfersystems aus Fig. 22;
- Fig. 25: eine isometrische Ansicht einer Rückseite des Beta-Behälters aus Fig. 24;
- Fig. 26: eine isometrische Ansicht des Beta-Behälters aus Fig. 24 ohne Abdeckelement;
- Fig. 27: eine isometrische Ansicht eines Funktionselements des Beta-Behälters aus Fig. 24;
- Fig. 28: eine isometrische Ansicht einer Rückseite des Funktionselements aus Fig. 27; und
- Fig. 29: eine Längsschnittansicht des Funktionselements aus Fig. 27.

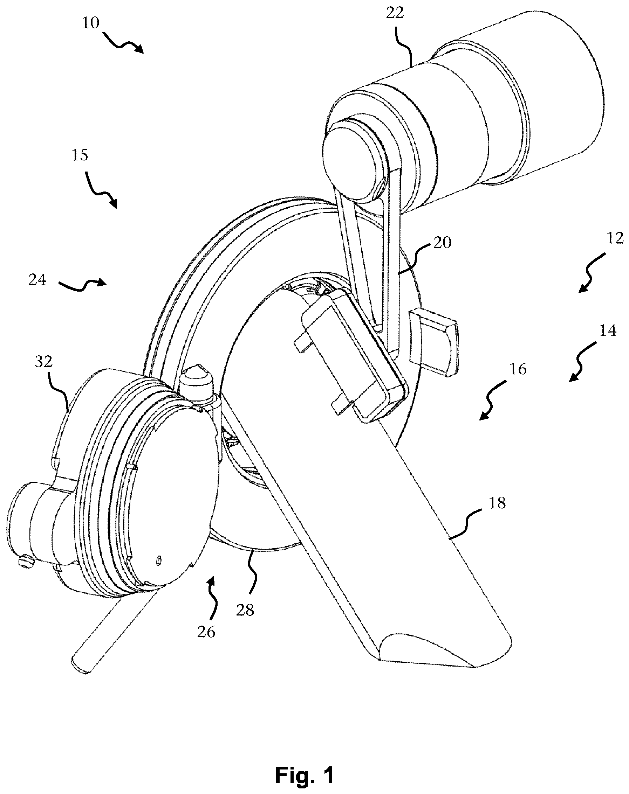

Die Figuren 1 bis 3 zeigen eine erste Ausführungsform eines Isolatorsystems als ein Barrieresystem in seiner Gesamtheit mit der Bezugsziffer 10 bezeichnet.

Das Isolatorsystem 10 weist einen Isolator 12 auf. Der Isolator 12 kann einen Innenraum 14 aufweisen. Der Isolator kann eine Isolatorwand (nicht dargestellt) aufweisen, die den Innenraum 14 umschließt bzw. umgibt. Die Isolatorwand trennt den Innenraum 14 von einer äußeren Umgebung 15, die den Isolator 12 umgibt. Der Isolator 12 ist vorzugsweise ein aseptischer Isolator.

Das Isolatorsystem 10 kann des Weiteren eine Zuführeinrichtung 16 aufweisen. Die Zuführeinrichtung 16 dient zum Zuführen von Objekten in den Innenraum des Isolators 12. Die Zuführeinrichtung 16 ist in dem Innenraum 14 des Isolators 12 angeordnet. Die Zuführeinrichtung 16 weist ein Rohr 18 auf. Die Zuführeinrichtung 16 weist einen Arm 20 auf. Ein erstes Ende des Arms 20 ist mit dem Rohr 18 fest verbunden. Ein zweites Ende des Arms 22 ist um eine horizontale Achse schwenkbar an der Isolatorwand gelagert. Die Zuführeinrichtung 16 weist eine Antriebseinrichtung 22 auf. Die Antriebseinrichtung 22 ist dazu eingerichtet, den Arm 20 gemeinsam mit dem Rohr 18 um die horizontale Achse zu schwenken. Das Rohr 18 weist ein erstes, offenes Ende 82 und ein zweites, offenes Ende 84 auf. Das Rohr 18 erstreckt sich von dem ersten Ende 82 zu dem zweiten Ende 84. Das Rohr 18 ist im Wesentlichen zylindrisch ausgebildet. Das Rohr 18 ist zumindest an dem ersten Ende 82 schräg abgeschnitten. Das Rohr 18 ist dadurch an dem ersten Ende 82 ellipsenförmig ausgebildet. Das Rohr 18 kann auch an den beiden Enden 82, 84 schräg abgeschnitten sein. Das Rohr 18 ist dann an beiden Enden 82, 84 ellipsenförmig ausgebildet.

Das Isolatorsystem 10 weist des Weiteren ein Transfersystem 24 auf. Das Transfersystem 24 dient zum Transfer von Objekten in den Isolator hinein bzw. Transfer von Objekten aus dem Isolator hinaus. Insbesondere können über das Transfersystem 24 Objekte, wie beispielsweise Verschlusselemente, in den Isolator eingebracht werden.

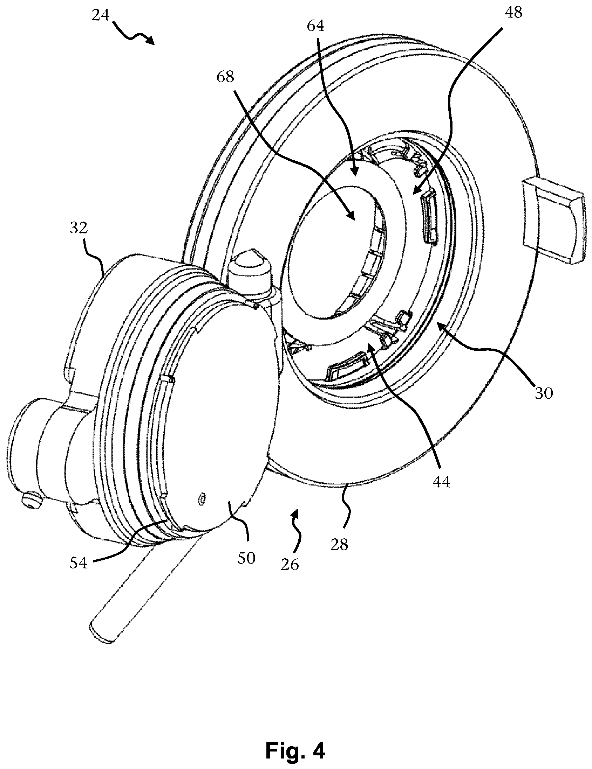

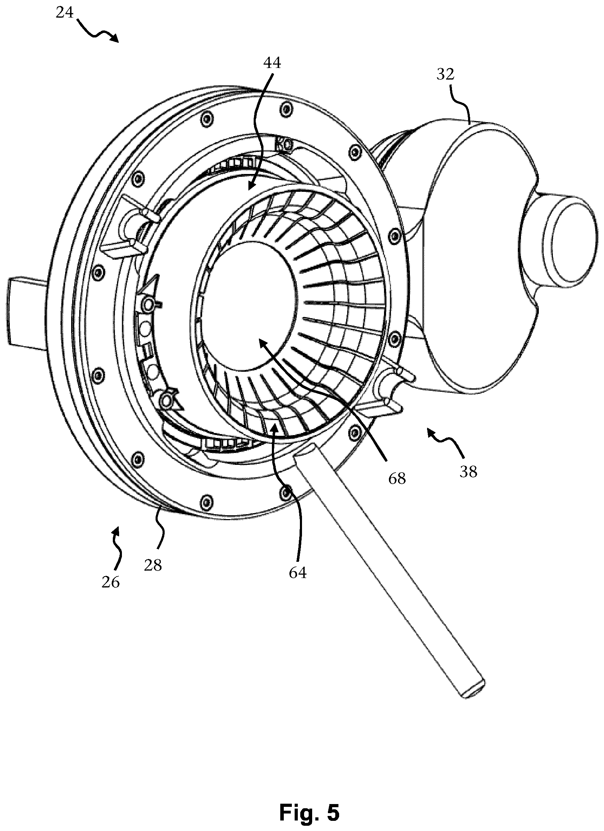

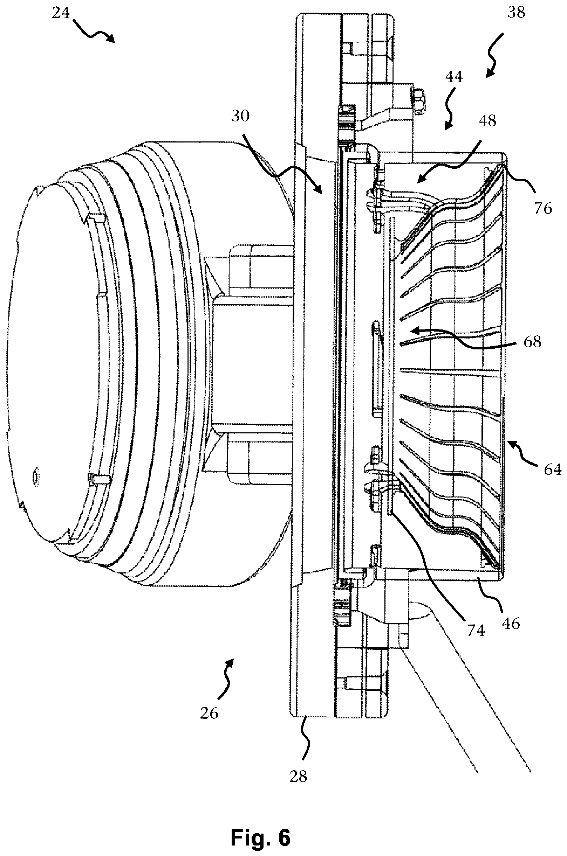

Das Transfersystem 24 ist in den Figuren 4 bis 6 detailliert dargestellt. Das Transfersystem weist einen Alpha-Port 26 und einen Beta-Behälter 38 auf. Der Alpha-Port 26 kann an der Isolatorwand des Isolators 12 angeordnet sein. Insbesondere ist der Alpha-Port 26 an einer vertikalen Wandfläche der Isolatorwand angeordnet. Der Alpha-Port 26 bildet insbesondere einen verschließbaren Durchgang durch die Isolatorwand von dem Innenraum 14 zu der äußeren Umgebung 15.

Die Zuführeinrichtung 16 ist von Innen an dem Alpha-Port 26 anordenbar. Wenn die Zuführeinrichtung 16 an dem Alpha-Port 26 angeordnet ist, können die Objekte aus dem Beta-Behälter 38 über die Zuführeinrichtung 16 in den Isolator 12 zugeführt werden.

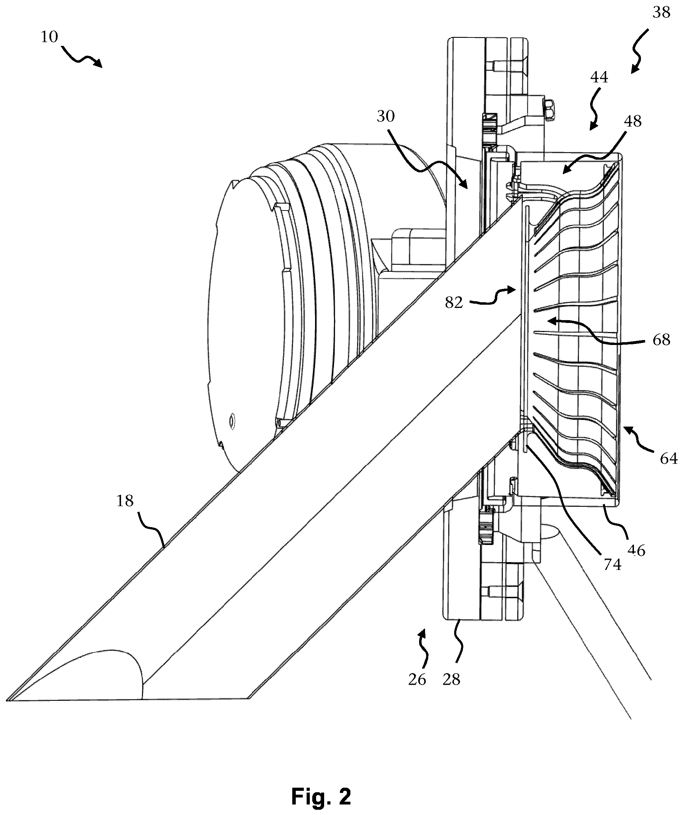

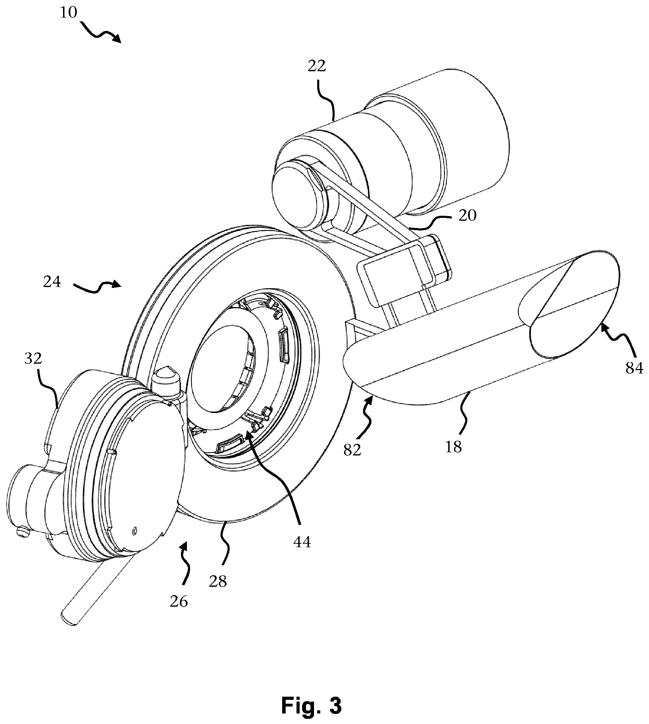

Die Zuführeinrichtung 16 ist vorzugsweise in einer vertikalen Richtung oberhalb des Alpha-Ports 26 schwenkbar angeordnet. Das Rohr 18 ist über den Arm 20 zwischen einer ersten Stellung und einer zweiten Stellung schwenkbar. In Fig. 1 und 2 ist das Rohr 18 in der ersten Stellung angeordnet. In der ersten Stellung ist das erste Ende 82 des Rohrs 18 an bzw. in dem Alpha-Port 26 angeordnet. Wenn das Rohr 18 in der ersten Stellung angeordnet ist, können Objekte aus dem Beta-Behälter 38 in das Rohr 18 eingeführt werden. In Fig. 3 ist das Rohr 18 in der zweiten Stellung angeordnet. In der zweiten Stellung ist das erste Ende 82 des Rohrs 18 nicht an dem Alpha-Port 26 angeordnet, sondern davon wegbewegt bzw. von dem Alpha-Port 26 beabstandet. Wenn das Rohr 18 in der zweiten Stellung angeordnet ist, kann die Tür 32 geöffnet und verschlossen werden.

In der ersten Stellung des Rohrs 18 ist das erste Ende 82 des Rohrs 18 in der vertikalen Richtung höher als das zweite Ende 84 angeordnet. Das Rohr 18 verläuft dadurch schräg zu einer horizontalen Richtung.

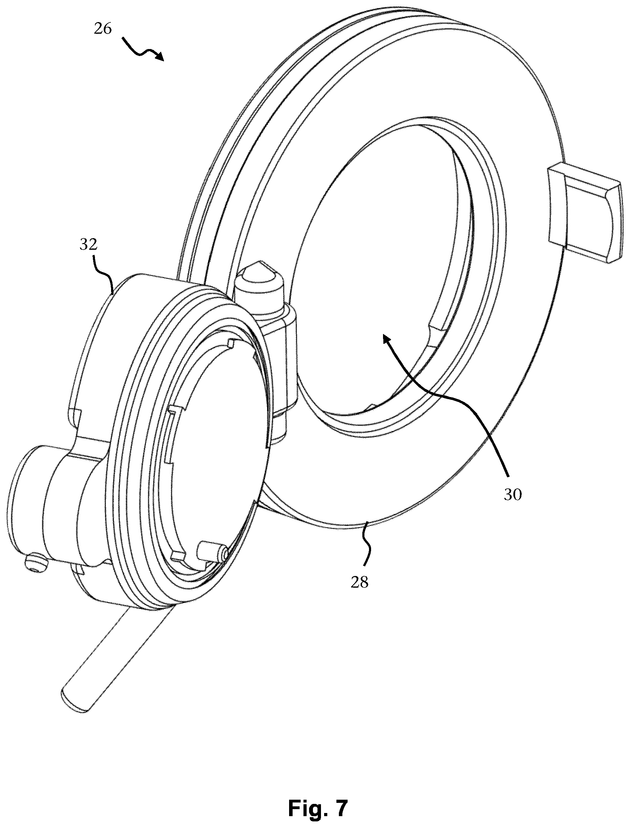

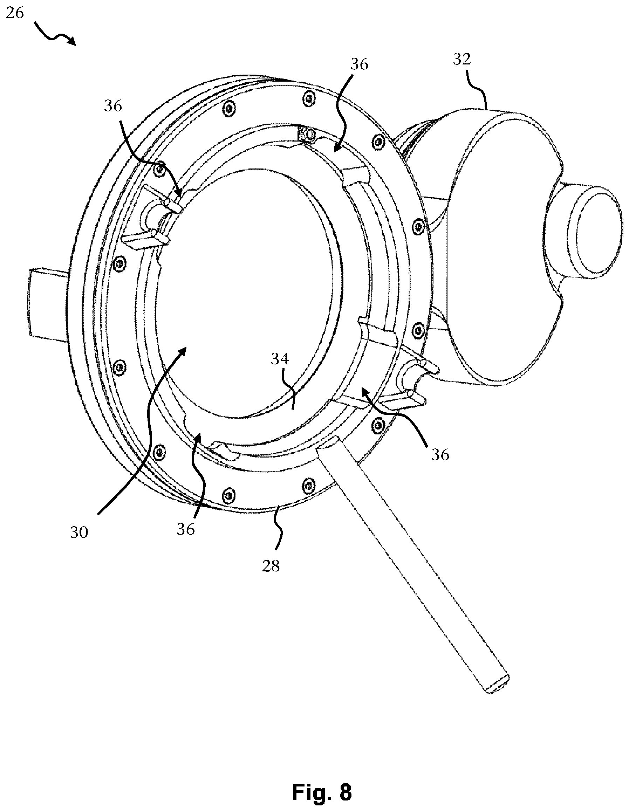

Der Alpha-Port 26 ist in den Figuren 7 und 8 detailliert dargestellt.

Der Alpha-Port 26 weist einen Alpha-Portgrundkörper 28 auf. Der Alpha-Portgrundkörper 28 kann ringförmig ausgebildet sein. Insbesondere kann sich der Alpha-Portgrundkörper 28 durch die Isolatorwand hindurch erstrecken. Der Alpha-Portgrundkörper 28 weist eine Innenseite und eine Außenseite auf. Die Innenseite ist dem Innenraum zugewandt. Die Außenseite ist der äußeren Umgebung 15 zugewandt. Im gekoppelten Zustand ist die Außenseite insbesondere dem Beta-Behälter 38 zugewandt

Der Alpha-Port 26 weist eine Alpha-Portöffnung 30 auf. Die Alpha-Portöffnung 30 bildet einen Durchgang durch die Isolatorwand. Die Alpha-Portöffnung 30 ist kreisförmig ausgebildet. Die Alpha-Portöffnung 30 kann durch eine Aussparung in dem Alpha-Portgrundkörper 28 gebildet sein, die sich von der Innenseite zu der Außenseite des Alpha-Portgrundkörpers 28 erstreckt. Der Alpha-Portgrundkörper 28 umgibt die die Alpha-Portöffnung 30.

Der Alpha-Port 26 weist des Weiteren eine Tür 32 auf. Die Tür 32 ist an der Alpha-Portöffnung 30 angeordnet. Die Tür 32 dient zum Öffnen und Verschließen der Alpha-Portöffnung 30. Die Tür 32 ist bewegbar, insbesondere schwenkbar, an dem Alpha-Portgrundkörper 28 angeordnet. Die Tür 32 kann bewegt werden, um die Alpha-Portöffnung 30 zu Öffnen oder zu Verschießen. In einem geschlossenen Zustand verschließt die Tür 32 die Alpha-Portöffnung 30 dicht. Zum Bewegen der Tür kann der Alpha-Port 26 beispielsweise eine Antriebseinrichtung (nicht dargestellt) aufweisen.

Der Alpha-Port 26 weist einen Alpha-Portflansch 34 auf. Der Alpha-Portflansch 34 ist an der Außenseite des Alpha-Portgrundkörpers 28 angeordnet. Insbesondere kann der Alpha-Portgrundkörper 28 den Alpha-Portflansch 34 auf der Außenseite ausbilden. Der Alpha-Portflansch 34 umgibt die Alpha-Portöffnung 30 an der Außenseite.

Der Alpha-Port 26 kann des Weiteren ein oder mehrere Kopplungselemente 36 aufweisen. Die Kopplungselemente 36 dienen zur Kopplung des Alpha-Ports 26 mit dem Beta-Behälter 38. Die Kopplungselemente 36 sind an dem Alpha-Portgrundkörpers 28, insbesondere an dem Alpha-Portflansch 34, angeordnet.

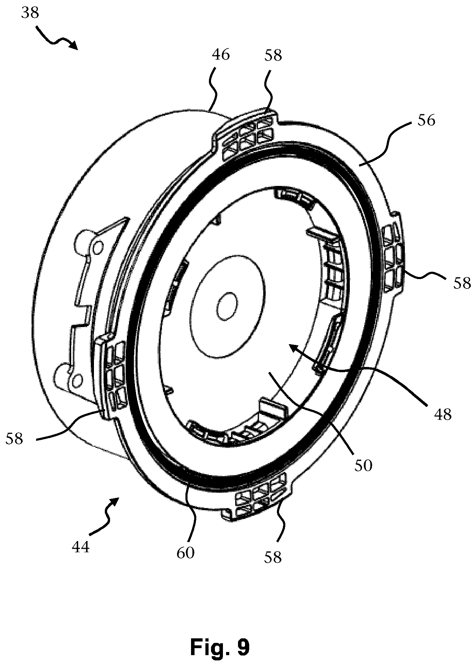

Der Beta-Behälter 38 ist in den Figuren 9 bis 11 detailliert dargestellt.

Der Beta-Behälter 38 weist eine Außenwand 40 auf. Die Außenwand 40 kann beispielsweise flexibel ausgestaltet sein. Insbesondere kann die Außenwand 40 ein flexibler Beutel sein. Der flexible Beutel kann aus Kunststoff bestehen. Der flexible Beutel kann beispielsweise ein Sterilbeutel sein. Alternativ kann die Außenwand 40 starr ausgebildet sein. Insbesondere kann die Außenwand 40 als ein starres Gehäuse ausgebildet sein. Das Gehäuse kann aus einem Kunststoff oder einem Metall, beispielsweise Aluminium oder Edelstahl, bestehen.

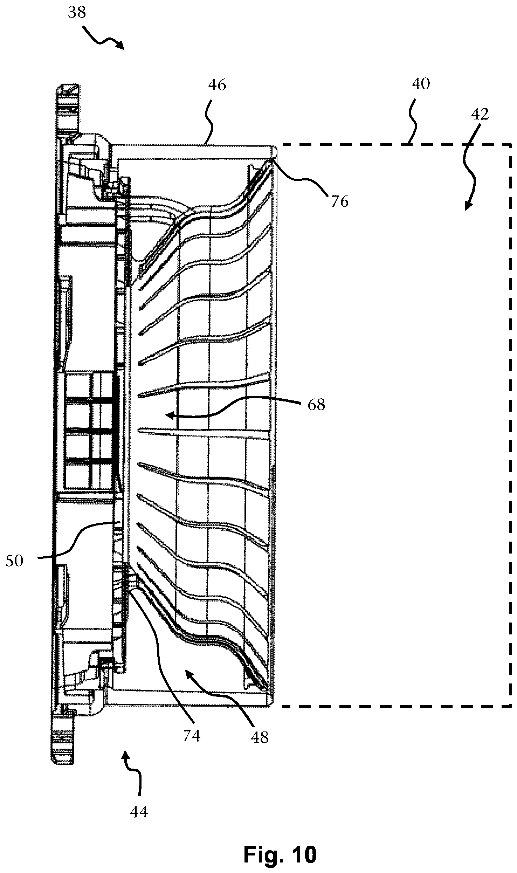

Der Beta-Behälter 38 weist des Weiteren ein Innenvolumen 42 auf. In dem Innenvolumen 42 können beispielsweise Objekte, wie Verschlusselemente, angeordnet werden. Die Außenwand 40 umgibt bzw. umschließt das Innenvolumen 42. Insbesondere isoliert die Außenwand 40 das Innenvolumen 42 gegenüber der äußeren Umgebung 15, die den Beta-Behälter 38 umgibt. Die Außenwand 40 und das Innenvolumen 42 sind in den Fig. 10 schematisch skizziert. In den übrigen Figuren des Isolatorsystems 10 der ersten Ausführungsform sind die Außenwand 40 und das Innenvolumen 42 zu Veranschaulichungszwecken nicht dargestellt.

Der Beta-Behälter 38 weist einen Beta-Port 44 auf. Der Beta-Port 44 kann an der Außenwand 40 des Beta-Behälters 38 angeordnet sein. Der Alpha-Port 26 und Beta-Port 44 sind miteinander koppelbar. Insbesondere kann der Beta-Port 44 von der äußeren Umgebung 15 an den Alpha-Port 26 gekoppelt werden. In einem gekoppelten Zustand des Alpha-Ports 26 und des Beta-Ports 44 ist das Innenvolumen 42 des Beta-Behälters 38 mit dem Innenraum 14 des Isolators 12 verbunden.

Der Beta-Port 44 weist einen Beta-Portgrundkörper 46 auf. Der Beta-Portgrundkörper 46 kann vorzugsweise ringförmig ausgebildet sein. Insbesondere kann sich der Beta-Portgrundkörper 46 durch die Außenwand 40 hindurch erstrecken. Der Beta-Portgrundkörper 46 weist eine Innenseite und eine Außenseite auf. Die Innenseite ist dem Innenvolumen 42 zugewandt. Die Außenseite ist der äußeren Umgebung 15 zugewandt. Im gekoppelten Zustand ist die Außenseite insbesondere dem Alpha-Port 26 zugewandt.

Der Beta-Port 44 weist eine Beta-Portöffnung 48 auf. Die Beta-Portöffnung 48 bildet einen Durchgang durch die Außenwand 40. Mit anderen Worten ist das Innenvolumen 42 durch die Beta-Portöffnung 48 von außen zugänglich. Die Beta-Portöffnung 48 ist kreisförmig, ausgebildet. Die Beta-Portöffnung 48 kann durch eine Aussparung in dem Beta-Portgrundkörper 46 gebildet sein, die sich von der Innenseite zu der Außenseite des Beta-Portgrundkörpers 46 erstreckt. Insbesondere umgibt der Beta-Portgrundkörper 46 die Beta-Portöffnung 48. Im gekoppelten Zustand sind die Alpha-Portöffnung 30 und die Beta-Portöffnung 48 konzentrisch zueinander angeordnet.

Die Beta-Portöffnung 48 und die Alpha-Portöffnung 30 sind im Wesentlichen gleich groß. Insbesondere können die Beta-Portöffnung 48 und die Alpha-Portöffnung 30 den gleichen Durchmesser aufweisen.

Der Beta-Port 44 weist einen Beta-Portflansch 56 auf. Der Beta-Portflansch 56 ist an der Außenseite des Beta-Portgrundkörpers 46 angeordnet. Insbesondere kann der Beta-Portgrundkörper 46 den Beta-Portflansch 56 auf der Außenseite ausbilden. Der Beta-Portflansch 56 umgibt die Beta-Portöffnung 48 an der Außenseite. Im gekoppelten Zustand liegen der Alpha-Portflansch 34 und der Beta-Portflansch 56umlaufend aneinander an.

Der Beta-Port 44 kann des Weiteren ein Dichtungselement 60 aufweisen. Das Dichtungselement 60 dient zum Abdichten der Kopplung zwischen dem Alpha-Port 26 und dem Beta-Port 44. Das Dichtungselement 60 kann auf der Außenseite des Beta-Portgrundkörpers 46 angeordnet sein. Insbesondere kann das Dichtungselement 60 an dem Beta-Portflansch 56 angeordnet sein. Insbesondere kann der Beta-Portflansch 56 auf der Außenseite eine um die Beta-Portöffnung 48 umlaufende Nut aufweisen, in die das Dichtungselement 60 eingelegt ist. Das Dichtungselement kann beispielsweise eine Lippendichtung sein. Im gekoppelten Zustand ist das Dichtungselement 60 zwischen dem Alpha-Portflansch 34 und dem Beta-Portflansch 56 angeordnet. Auf diese Weise werden das Innenvolumen 42 des Beta-Behälters 38 und der Innenraum 14 des Isolators 12 gegenüber der äußerten Umgebung 15 abgedichtet.

Der Beta-Port 44 weist ein Abdeckelement 50 auf. Das Abdeckelement 50 ist an bzw. in der Beta-Portöffnung 48 anordenbar. Das Abdeckelement 50 dient zum Abdecken bzw. Verschließen der Beta-Portöffnung 48.

Wenn das Abdeckelement 50 an bzw. in der Beta-Portöffnung 48 angeordnet ist und die Beta-Portöffnung 48 abdeckt, ist der Beta-Behälters 38 geschlossen. Der Beta-Behälters 38 befindet sich dann in einem geschlossenen Zustand. In Fig. 9 ist der Beta-Behälter 38 im geschlossenen Zustand dargestellt.

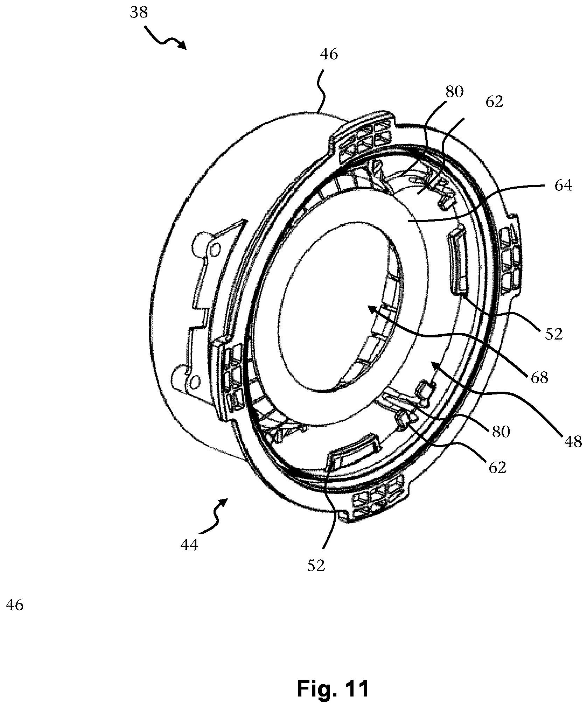

Wenn das Abdeckelement 50 nicht an bzw. in der Beta-Portöffnung 48 angeordnet ist und die Beta-Portöffnung 48 nicht abdeckt, insbesondere wenn das Abdeckelement 50 von der Beta-Portöffnung 48 abgenommen ist, ist der Beta-Behälters 38 geöffnet. Der Beta-Behälters 38 befindet sich dann in einem geöffneten Zustand. In Fig. 11 ist der Beta-Behälter 38 ohne das Abdeckelement 50 im geöffneten Zustand dargestellt.

Das Abdeckelement 50 kann lösbar mit dem Beta-Portgrundkörper 46 oder dem Beta-Portflansch 56 koppelbar sein. Wenn das Abdeckelement 50 mit dem Beta-Portgrundkörper 46 oder dem Beta-Portflansch 56 gekoppelt ist, verschließt das Abdeckelement 50 die Beta-Portöffnung 48. Zur Kopplung können das Abdeckelement 50 und der Beta-Portgrundkörper 46 korrespondierende Kopplungselemente aufweisen. Insbesondere kann der Beta-Portgrundkörper 46 in der Beta-Portöffnung 48 Vorsprünge 52 und das Abdeckelement dazu entsprechende Aufnahmen 54 für die Vorsprünge 52 aufweisen. Zum Befestigen des Abdeckelements 50 an bzw. in der Beta-Portöffnung 48 können die Vorsprünge 52 in Eingriff mit den Aufnahmen gebracht werden.

Der Beta-Port 44 kann des Weiteren ein oder mehrere Kopplungselemente 58 aufweisen. Die Kopplungselemente 58 dienen zur Kopplung des Beta-Ports 44 mit dem Alpha-Port 26. Insbesondere können die Kopplungselemente 58 des Beta-Ports mit den Kopplungselementen 36 des Alpha-Ports koppeln, um den Alpha-Port und den beta-Port miteinander zu koppeln. Die Kopplungselemente 58 sind an dem Beta-Portgrundkörpers 46, insbesondere an dem Beta-Portflansch 56, angeordnet.

Die Tür 32 und das Abdeckelement 50 sind miteinander koppelbar. Wenn die Tür 32 und das Abdeckelement 50 gekoppelt sind, lassen sich die Tür 32 und das Abdeckelement 50 gemeinsam bewegen. Insbesondere können diese derart bewegt werden, dass die Alpha-Portöffnung 30 und die Beta-Portöffnung 48 gemeinsam geöffnet und verschlossen werden können. Insbesondere können die Tür 32 und das Abdeckelement 50 miteinander koppeln, wenn der Alpha-Port 26 und der Beta-Port 44 miteinander gekoppelt werden. Dadurch können Alpha-Portöffnung 30 und die Beta-Portöffnung 48 gemeinsam geöffnet und verschlossen werden, wenn der Alpha-Port 26 und der Beta-Port 44 miteinander gekoppelt sind.

Zur Kopplung können die Tür 32 und das Abdeckelement 50 korrespondierende Kopplungselemente aufweisen, wobei ein oder mehrere Kopplungselemente der Tür 32 mit einem oder mehreren Kopplungselementen des Abdeckelements 50 koppelbar sind.

In dem Beta-Behälter 38 können Objekte, wie beispielsweise Verschlusselemente, bereitgestellt sein, die in den Isolator 12 transferiert werden sollen. Die Objekte können dann im gekoppelten Zustand des Alpha-Ports 26 und des Beta-Ports 44 in den Isolator 12 transferiert bzw. eingebracht werden. Insbesondere können die Objekte aus dem Beta-Behälter durch den Beta-Port 44, den Alpha-Port 26 und die Zuführeinrichtung 16 in den Isolator zugeführt werden.

Der Beta-Behälter 38 weist des Weiteren ein Funktionselement 64 auf. Das Funktionselement dient dazu, den Transfer von Objekten zwischen dem Beta-Behälter und dem Isolator zu führen. Das Funktionselement 64 ist vorzugsweise in dem Beta-Behälter 38 anordenbar. Das Funktionselement 64 ist an und/oder in der Beta-Portöffnung 48 anordenbar. Vorzugsweise kann das Funktionselement 64 zumindest teilweise in der Beta-Portöffnung 48 angeordnet sein. Das Funktionselement ist insbesondere an dem Beta-Portgrundkörper 46 anordenbar.

Das Funktionselement 64 ist in dieser Ausführungsform als ein Einsatzelement ausgebildet, das in den Beta-Behälters 38, insbesondere in die Beta-Portöffnung 48, einsetzbar ist. Insbesondere kann das Funktionselement 64 an dem Beta-Portgrundkörper 46, vorzugsweise lösbar, befestigbar sein. Im eingesetzten Zustand ist das Funktionselement 64 an dem Beta-Portgrundkörper befestigt.

Im geschlossenen Zustand des Beta-Behälters 38 kann das Funktionselement 64 zwischen dem Abdeckelement 50 und dem Innenvolumen 42 angeordnet sein. Insbesondere ist das Funktionselement 64 an dem oder angrenzend an das Abdeckelement 50 angeordnet.

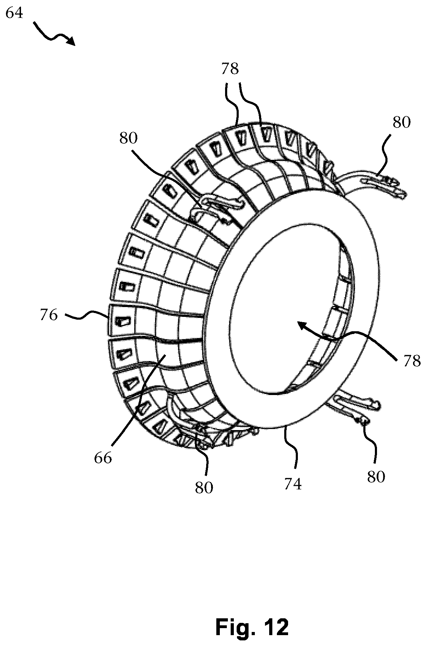

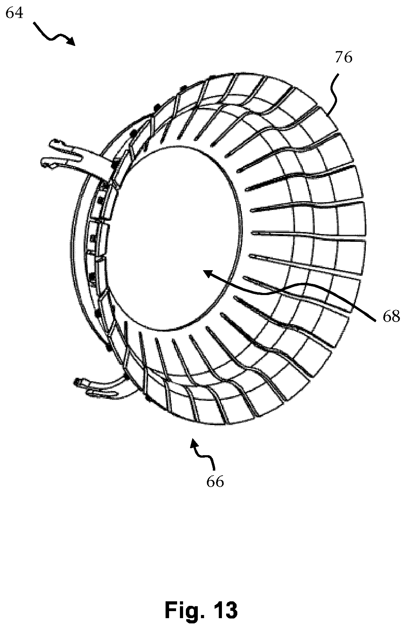

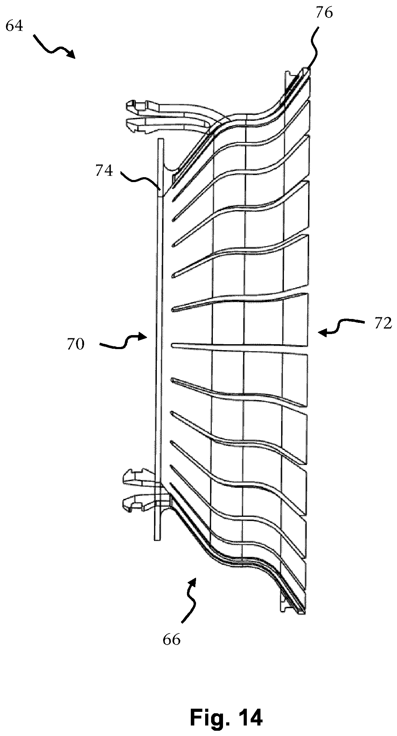

Das Funktionselement 64 ist in den Figuren 12 bis 14 detailliert dargestellt.

Das Funktionselement 64 weist einen Funktionselementkörper 66 auf. Der Funktionselementkörper 66 weist einen äußeren Rand 76 auf. Der äußere Rand 76 ist an dem Beta-Portgrundkörper angeordnet. Insbesondere liegt der äußere Rand 76 umlaufend an dem Beta-Portgrundkörper 46 in der Beta-Portöffnung 48 an, wenn das Funktionselement 64 zumindest teilweise in der Beta-Portöffnung 48 angeordnet bzw. eingesetzt ist. Der äußere Rand 76 kann vorzugsweise komplementär zu der Beta-Portöffnung 48 ausgebildet sein. Insbesondere kann der äußere Rand 76 in der Beta-Portöffnung 48 umlaufend an dem Beta-Portgrundkörper46 angeordnet sein bzw. an diesem umlaufend anliegen.

Das Funktionselement 64 weist eine Funktionselementöffnung 68 auf. Die Funktionselementöffnung 68 ist kreisförmig ausgebildet. Die Funktionselementöffnung 68 ist kleiner als die Beta-Portöffnung 48. Insbesondere ist ein Durchmesser der Funktionselementöffnung 68 kleiner als ein Durchmesser der Beta-Portöffnung 48. Die Funktionselementöffnung 68 ist konzentrisch zu der Beta-Portöffnung 48 anordenbar. Insbesondere ist die Funktionselementöffnung 68 im eingesetzten Zustand konzentrisch zu der Beta-Portöffnung 48 angeordnet.

Die Funktionselementöffnung 68 ist als eine Aussparung in dem Funktionselementkörper 66 ausgebildet. Die Funktionselementöffnung 68 erstreckt sich in einer axialen Richtung durch den Funktionselementkörper 66 hindurch. Der Funktionselementkörper 66 umgibt die Funktionselementöffnung 68. Der Funktionselementkörper 66 erstreckt sich von der Funktionselementöffnung 68 radial nach außen. Der Funktionselementkörper 66 ist vorzugsweise ringförmig oder trichterförmig ausgebildet.

Das Funktionselement 64 erstreckt sich in der axialen Richtung von einem ersten, axialen Ende 70 und einem zweiten, axialen Ende 72. Im eingesetzten Zustand sind das zweite, axiale Ende 72 dem Innenvolumen 42 zugewandt und das erste, axiale Ende 70 dem Innenvolumen 42 abgewandt. Wenn der Beta-Port 44 mit dem Alpha-Port 26 gekoppelt ist, ist das erste, axiale Ende 70 dem Alpha-Port 26 zugewandt und das zweite, axiale Ende 72 dem Alpha-Port 26 abgewandt.

Das Funktionselement 64 weist einen Funktionselementflansch 74 auf. Der Funktionselementkörper weist den Funktionselementflansch 74 an dem ersten, axialen Ende 70 auf. Der Funktionselementflansch 74 umgibt die Funktionselementöffnung 68 an dem ersten, axialen Ende 70. Im eingesetzten Zustand ist das Funktionselement 64 derart in der Beta-Portöffnung 48 angeordnet, dass der Funktionselementflansch 74 angrenzend an dem Abdeckelement 50 angeordnet ist, wenn die Beta-Portöffnung mit Abdeckelement 50 abgedeckt bzw. verschlossen ist.

Der äußere Rand 76 ist an dem zweiten, axialen Ende 72 angeordnet. Der äußere Rand 76 ist in der radialen Richtung radial Außen angeordnet. Insbesondere ist der äußere Rand 76 radial weiter außen angeordnet als der Funktionselementflansch 74.

Der äußere Rand 76 ist elastisch, insbesondere lamellenartig, ausgebildet. Dazu kann der Funktionselementkörper 66 umlaufend eine Mehrzahl von Lamellen aufweisen, die sich jeweils bis zu dem äußeren Rand 76 erstrecken.

Das Funktionselement 64 kann ein oder mehrere Befestigungselemente 80 aufweisen, mittels denen das Funktionselement 64 an dem Beta-Port befestigbar ist. Die Befestigungselemente 80 können hakenförmig ausgebildet sein. Vorzugsweise sind die Befestigungselemente 80 an dem äußeren Rand 76 des Funktionselementkörpers 66 angeordnet. Der Beta-Port 44 kann ein oder mehrere korrespondierende Aufnahmeelemente 62 aufweisen. Die Aufnahmeelemente 62 sind an dem Beta-Portgrundkörper 46 in der Beta-Portöffnung 48 angeordnet. Die Befestigungselemente 80 können in Eingriff mit den Aufnahmeelementen 62 gebracht werden, um das Funktionselement 64 zu befestigen. Je ein Befestigungselement 80 kann dabei in Eingriff mit einem entsprechenden Aufnahmeelement 62 gebracht werden.

Wie zuvor bereits beschrieben, können in dem Innenvolumen 42 des Beta-Behälters 38 Objekte, wie beispielsweise Verschlusselemente, bereitgestellt werden, wobei diese Objekte dann mittels des Transfersystems 24 und der Zuführeinrichtung 16 in den Innenraum des Isolators 12 eingebracht bzw. zugeführt werden können. Dazu wird zunächst der Beta-Port 44 mit dem Alpha-Port 26 gekoppelt und die Tür 32 gemeinsam mit dem Abdeckelement 50 geöffnet. Dann kann das Rohr 18 der Zuführeinrichtung 16 in die erste Stellung geschwenkt werden. Dieser erreichte Zustand ist in den Figuren 1 und 2 dargestellt. Die Objekte können dann aus dem Innenvolumen 42 durch die Beta-Portöffnung 48, die Alpha-Portöffnung 30 und das Rohr 18 in den Isolator 12 zugeführt werden. Das Funktionselement 64 ist dabei derart angeordnet, dass die Objekte durch die Funktionselementöffnung 68 hindurchmüssen, wenn die Objekte die Beta-Portöffnung 48 passieren.

Da der Alpha-Port 26 an einer vertikalen Wandfläche der Isolatorwand angeordnet ist, erstreckt sich die Alpha-Portöffnung 30 in einer horizontalen Richtung von der Innenseite zu der Außenseite des Alpha-Portgrundkörpers 28. Das Rohr 18 ist in der ersten Stellung schräg zu der horizontalen Richtung angeordnet. Dadurch ist das Rohr 18 in der ersten Stellung schräg zu der Alpha-Portöffnung 30 ausgerichtet.

In der ersten Stellung des Rohrs 18 erstreckt sich das erste Ende des Rohrs 82 in die Alpha-Portöffnung 30 hinein bis zu der Beta-Portöffnung 48. Dabei erstreckt sich das erste Ende des Rohrs 82 insbesondere bis zu dem Funktionselementflansch 74. Das erste Ende des Rohrs 82 liegt an dem Funktionselementflansch 74 an. Insbesondere ist die Funktionselementöffnung 68 derart ausgebildet, dass diese kleiner oder gleich als die Öffnung des Rohrs 18 an dem ersten Ende 82 ist. Die Funktionselementöffnung 68 ist fluchtend mit der Öffnung des Rohrs 18 an dem ersten Ende 82 angeordnet. Dadurch gehen alle Objekte, die die Funktionselementöffnung 68 an dem ersten axialen Ende 70 verlassen, direkt in das Rohr 18 hinein.

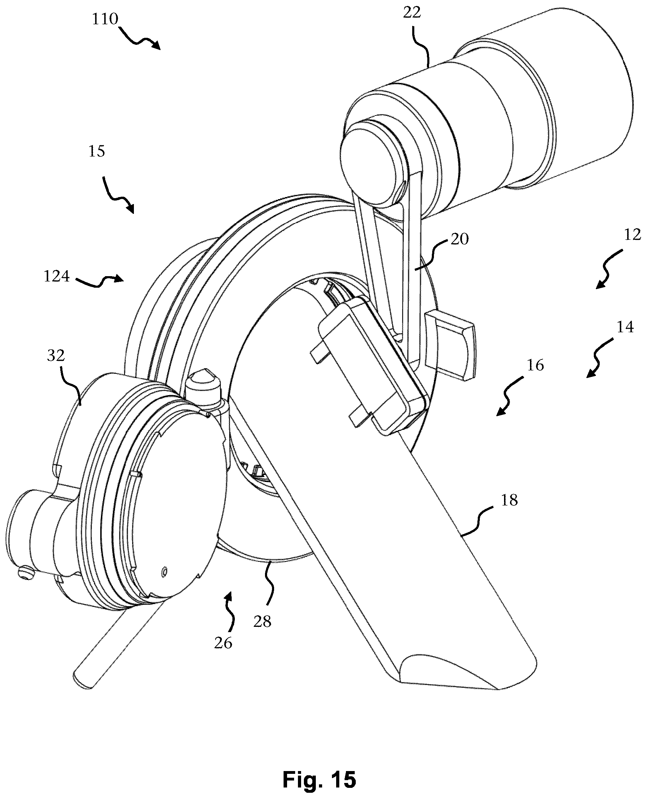

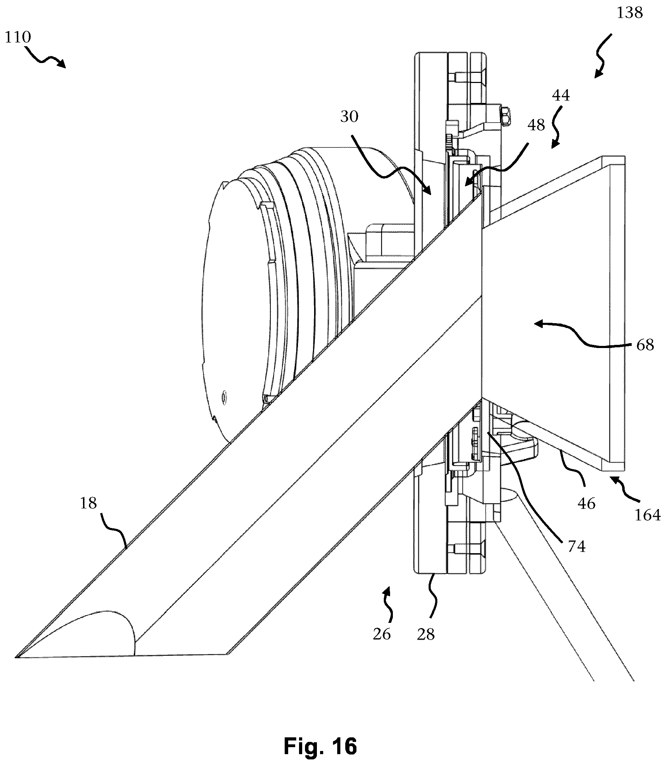

Die Figuren 15 und 16 zeigen eine zweite Ausführungsform eines Isolatorsystems als ein Barrieresystem in seiner Gesamtheit mit der Bezugsziffer 110 bezeichnet. Das Isolatorsystem 110 der zweiten Ausführungsform entspricht im Wesentlichen dem Isolatorsystem 10 der ersten Ausführungsform. Gleiche Elemente sind mit den gleichen Bezugszeichen gekennzeichnet und werden im Folgenden nicht näher erläutert. Das Isolatorsystem 110 unterscheidet sich von dem Isolatorsystem 10 in der Ausgestaltung des Transfersystems.

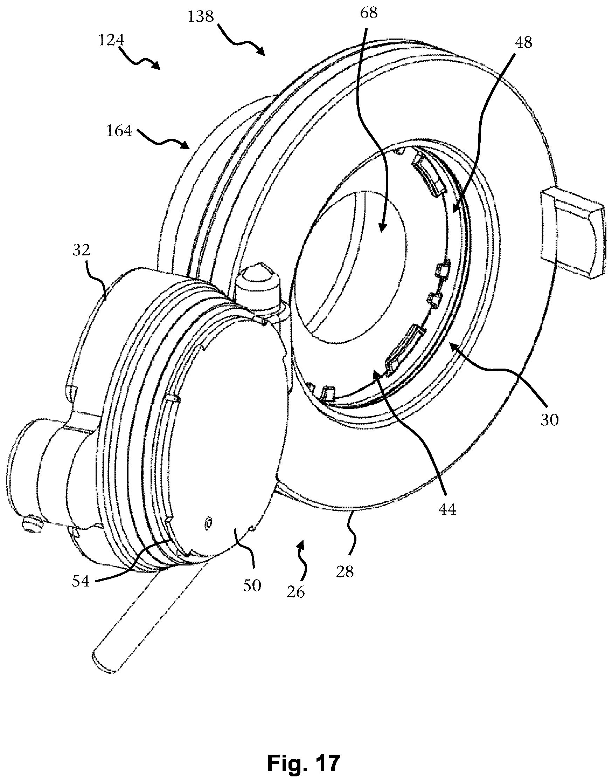

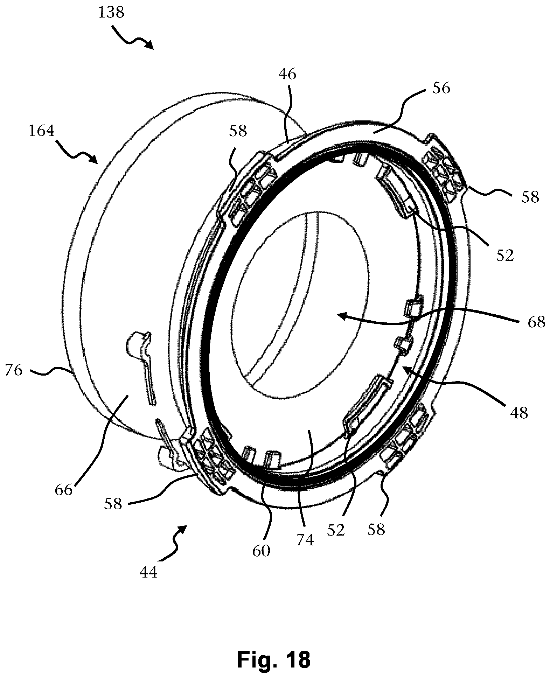

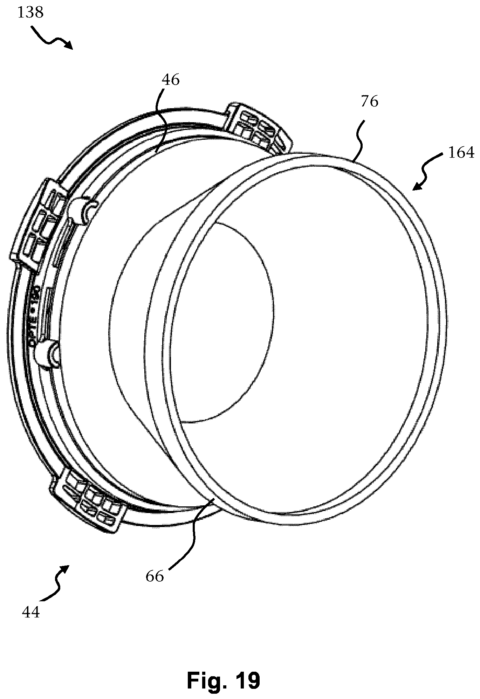

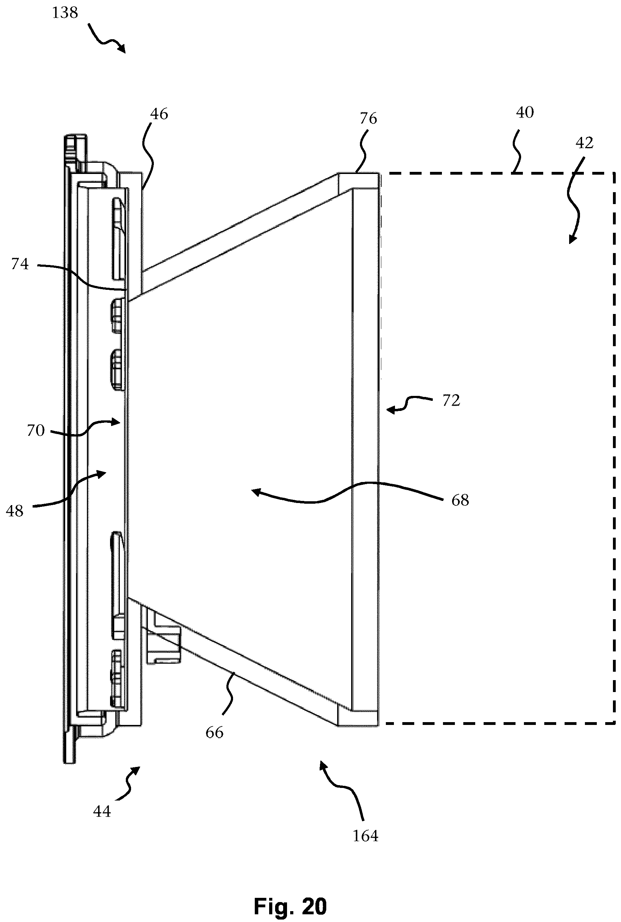

Das Transfersystem des Isolatorsystem 110 ist mit der Bezugsziffer 124 bezeichnet. Das Transfersystem 124 ist in Fig. 17 dargestellt. Das Transfersystem 124 weist den gleichen Alpha-Port 26 wie das Transfersystem 24 des Isolatorsystems 10 auf. Das Transfersystem 124 weist des Weiteren einen Beta-Behälter 138 auf. Der Beta-Behälter 138 hat im Wesentlichen den gleichen Aufbau, wie der Beta-Behälter 38. Der Beta-Behälter 138 unterscheidet sich von dem Beta-Behälter 38 in der Ausgestaltung des Funktionselements. Der Beta-Behälter 138 ist in den Figuren 18 bis 20 dargestellt.

Das Funktionselement des Beta-Behälters 138 ist mit der Bezugsziffer 164 bezeichnet. Das Funktionselement 164 ist einstückig mit dem Beta-Portgrundkörper 46 ausgebildet. Insbesondere ist das Funktionselement 164 in dieser Ausführungsform nicht als Einsatzelement ausgebildet. Entsprechend weist der Beta-Port keine Aufnahmeelemente 62 und das Funktionselement 164 weist keine Befestigungselemente 80 auf.

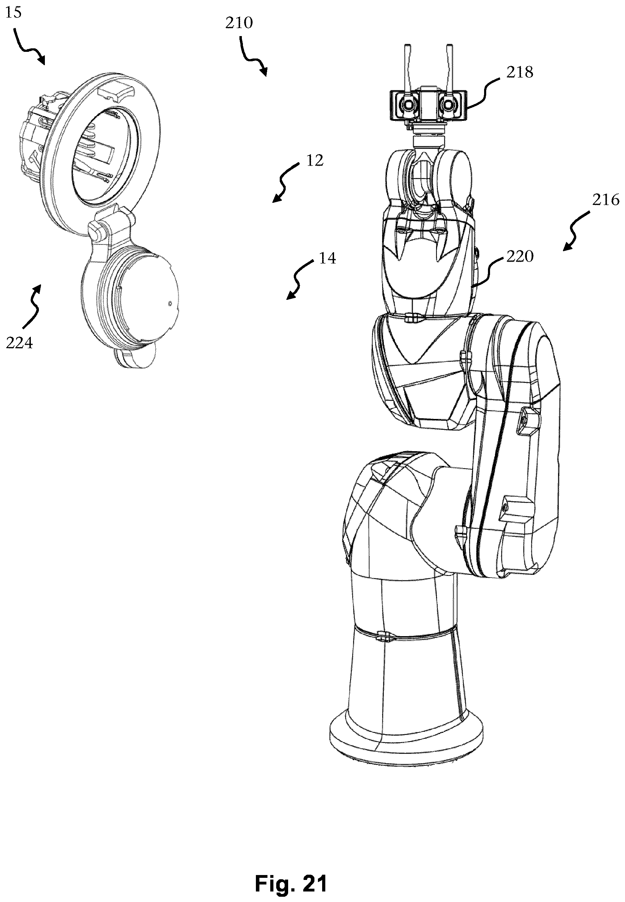

Die Fig. 21 zeigt eine dritte Ausführungsform eines Isolatorsystems als ein Barrieresystem in seiner Gesamtheit mit der Bezugsziffer 210 bezeichnet. Das Isolatorsystem 210 der zweiten Ausführungsform hat einen ähnlichen Aufbau wie das Isolatorsystem 10 der ersten Ausführungsform. Gleiche Elemente sind mit den gleichen Bezugszeichen gekennzeichnet und werden im Folgenden nicht näher erläutert.

In der dritten Ausführungsform weist das Isolatorsystem 210 in der dargestellten Form keine Zuführeinrichtung 16 auf.

Das Isolatorsystem 210 weist eine Handhabungseinrichtung 216 innerhalb des Isolators 12 auf. Die Handhabungseinrichtung 216 dient zum Handhaben, insbesondere zum Transferieren, von Nährbodenträgern. Die Handhabungseinrichtung 216 weist einen Endeffektor 218 auf. Der Endeffektor kann ein Greifwerkzeug mit beispielsweise einem Greifer aufweisen. Mit dem Greifwerkzeug können Nährbodenträger gegriffen und gehalten werden. Die Handhabungseinrichtung 216 weist einen mehrachsigen Arm 220 auf. Der Endeffektor 218 ist an einem Ende des Arms 220 angeordnet. Der Endeffektor 218 kann mittels des Arms 220 im Isolator 12 bewegt werden. Zum Bewegen des Arms kann die Handhabungseinrichtung 216 beispielsweise eine Antriebseinrichtung aufweisen.

Das Isolatorsystem 210 der dritten Ausführungsform unterscheidet sich von dem Isolatorsystem 10 der ersten Ausführungsform des Weiteren in der Ausgestaltung des Transfersystems.

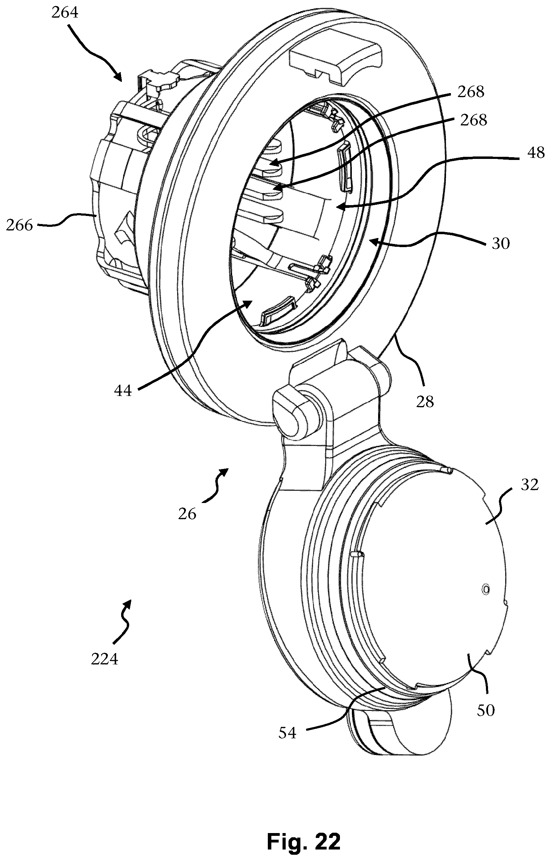

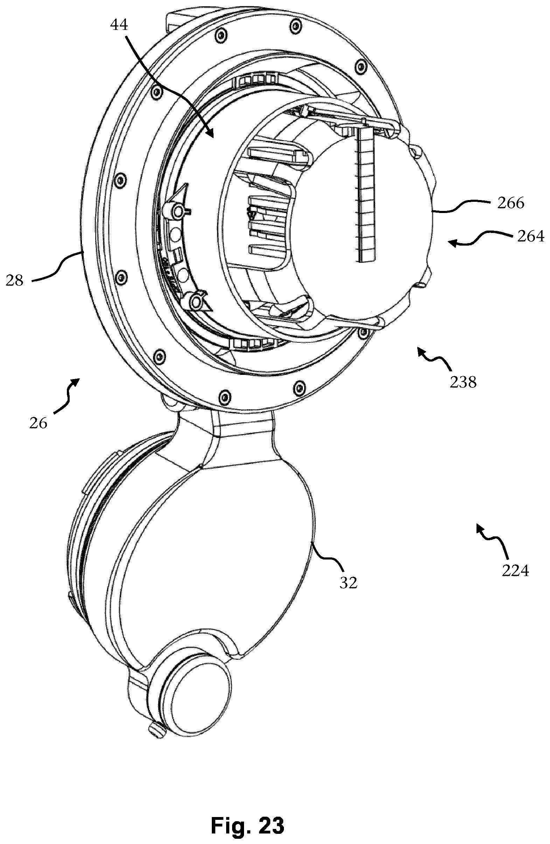

Das Transfersystem des Isolatorsystem 210 ist mit der Bezugsziffer 224 bezeichnet. Das Transfersystem 224 ist in den Figuren 22 und 23 dargestellt. Das Transfersystem 224 dient zum Transfer von Nährbodenträgern in den Isolator hinein oder aus dem Isolator hinaus.

Das Transfersystem 224 weist den gleichen Alpha-Port 26 wie das Transfersystem 24 des Isolatorsystems 10 auf. In dem Transfersystem 224 ist der Alpha-Port 26 gedreht angeordnet, so dass die Tür 32 des Alpha-Port 26 an dem Alpha-Portgrundkörper 28 nicht seitlich neben der Alpha-Portöffnung 30, sondern unterhalb der Alpha-Portöffnung 30 angeordnet ist.

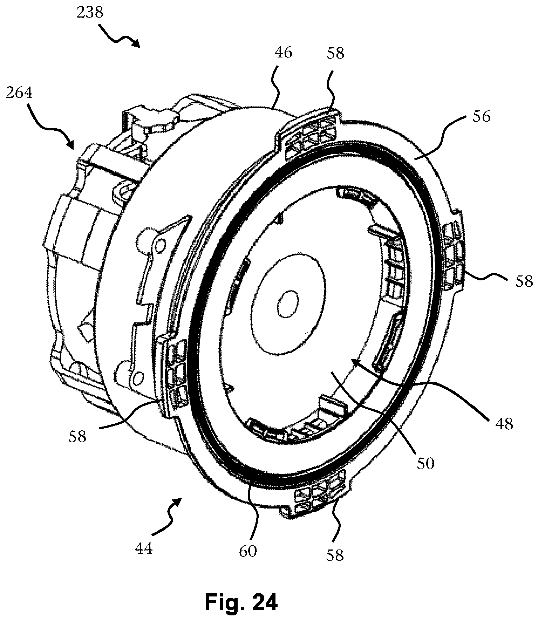

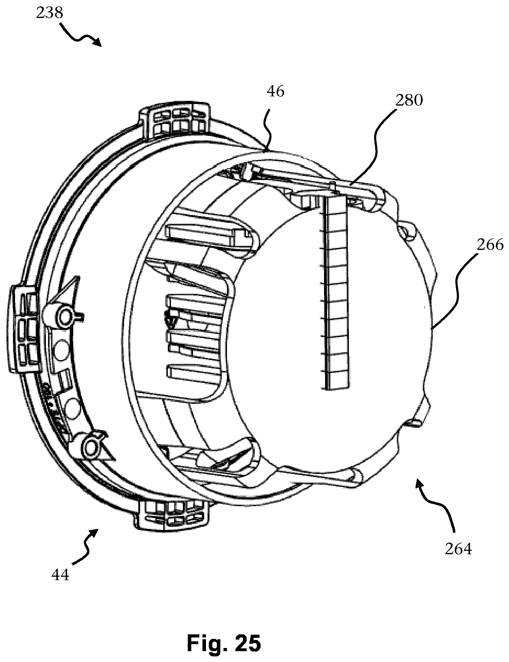

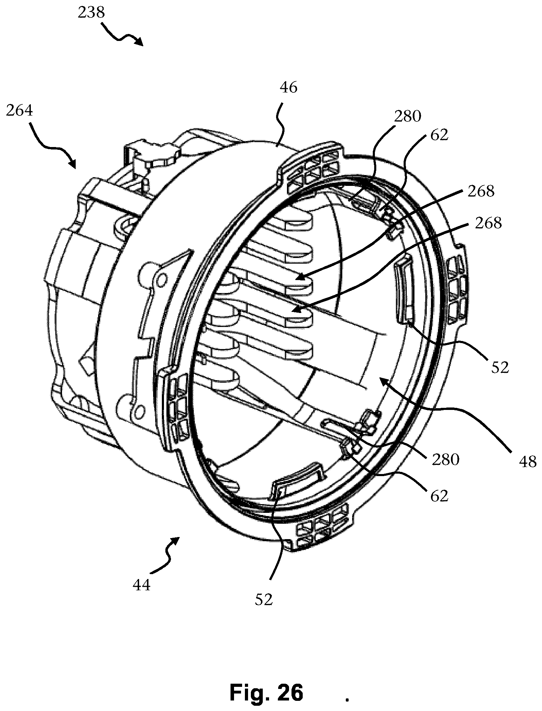

Das Transfersystem 224 weist des Weiteren einen Beta-Behälter 238 auf. Der Beta-Behälter 238 hat im Wesentlichen den gleichen Aufbau, wie der Beta-Behälter 38. Der Beta-Behälter 238 unterscheidet sich von dem Beta-Behälter 38 in der Ausgestaltung des Funktionselements. Der Beta-Behälter 138 ist in den Figuren 24 bis 26 dargestellt. In Fig. 24 ist der Beta-Behälter 238 im geschlossenen Zustand dargestellt. In Fig. 26 ist der Beta-Behälter 238 ohne das Abdeckelement 50 im geöffneten Zustand dargestellt.

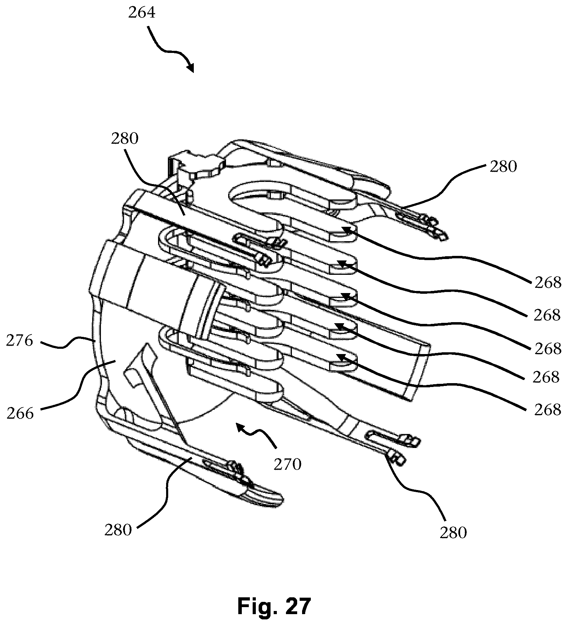

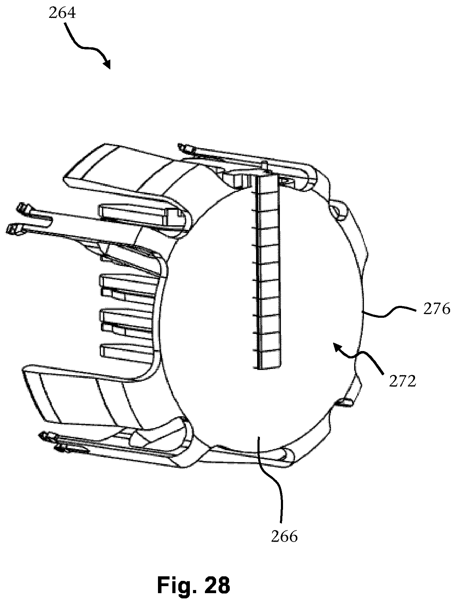

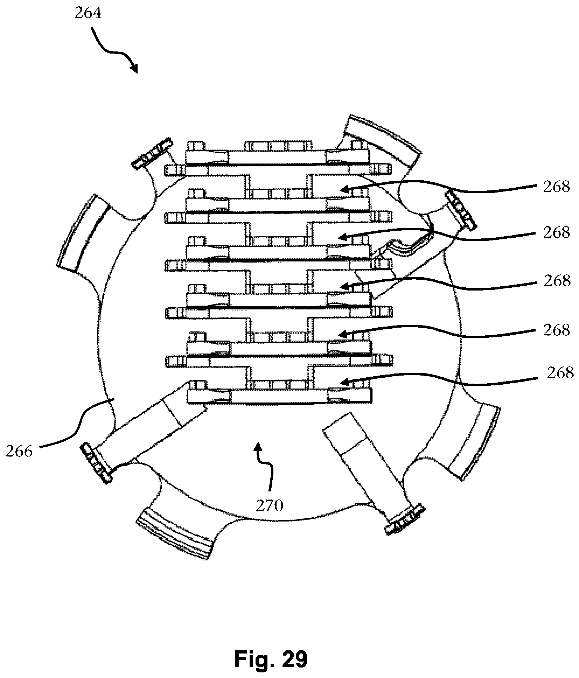

Das Funktionselement des Beta-Behälters 238 ist mit der Bezugsziffer 264 bezeichnet. Das Funktionselement 264 weist einen anderen Aufbau als das Funktionselement 38 der ersten Ausführungsform auf. Das Funktionselement 264 ist in den Figuren 27 bis 29 dargestellt. Das Funktionselement 264 dient zum Transfer von einem oder mehreren Nährbodenträgern in den Isolator hinein oder aus dem Isolator hinaus.

Das Funktionselement 264 ist vorzugsweise in dem Beta-Behälter 238 anordenbar. Das Funktionselement 264 ist insbesondere an und/oder in der Beta-Portöffnung 48 anordenbar. In der dargestellten Ausführungsform ist das Funktionselement 264 zumindest teilweise in der Beta-Portöffnung 48 anordenbar.

Das Funktionselement 264 ist in dieser Ausführungsform als ein Einsatzelement ausgebildet, das in den Beta-Behälters 238, insbesondere in die Beta-Portöffnung 48, einsetzbar ist. Insbesondere kann das Funktionselement 264 an dem Beta-Portgrundkörper 46, vorzugsweise lösbar, befestigbar sein. Insbesondere ist das Funktionselement 264 im eingesetzten Zustand an dem Beta-Portgrundkörper 46 befestigt.

Im geschlossenen Zustand des Beta-Behälters 238 kann das Funktionselement 264 zwischen dem Abdeckelement 50 und dem Innenvolumen 42 anordenbar sein. Insbesondere kann das Funktionselement 264 in dem Innenvolumen 42 angeordnet sein und sich aus dem Innenvolumen 42 in die Beta-Portöffnung 48 hinein erstrecken.

Das Funktionselement 264 weist einen Funktionselementkörper 266 auf. Das Funktionselement 264 weist des Weiteren einen oder mehrere Nährbodenträgerhalter 268 auf. Die einen oder mehrere Nährbodenträgerhalter 268 sind an dem Funktionselementkörper 266 angeordnet. Insbesondere sind die Nährbodenträgerhalter 268 an dem Funktionselementkörper 266 befestigt oder werden durch diesen ausgebildet.

Jeder Nährbodenträgerhalter 268 ist dazu eingerichtet, einen Nährbodenträger zu halten. Insbesondere kann in jedem Nährbodenträgerhalter 268 ein Nährbodenträger aufgenommen werden. Jeder Nährbodenträgerhalter kann beispielsweise ein Aufnahmeelement oder eine Auflagefläche für jeweils einen Nährbodenträger aufweisen.

Der Funktionselementkörper 266 kann im Wesentlichen scheibenförmig ausgebildet sein. Der Funktionselementkörper 266 weist eine erste Seite 270 und eine entgegengesetzte, zweite Seite 272 auf. Die Nährbodenträgerhalter 268 sind auf der ersten Seite 270 des Funktionselementkörper 266 angeordnet. Der Funktionselementkörper 266 weist einen äußeren, umlaufenden Rand 276 auf.

Im eingesetzten Zustand ist der Funktionselementkörper 266 in dem Innenvolumen 42 angeordnet. Im eingesetzten Zustand erstrecken sich die Nährbodenträgerhalter 268 von dem Funktionselementkörper 266 in die Beta-Portöffnung 48 hinein. Die erste Seite 270 ist im eingesetzten Zustand der Beta-Portöffnung 48 zugewandt. Die zweite Seite 272 ist im eingesetzten Zustand der Beta-Portöffnung 48 abgewandt. Im gekoppelten Zustand des Alpha-Ports 26 und des Beta-Ports 44 ist die erste Seite 270 somit dem Alpha-Port 26 zugewandt.

Das Funktionselement 264 kann ein oder mehrere Befestigungselemente 280 aufweisen, mittels denen das Funktionselement 264 an dem Beta-Port 44 befestigbar ist. Die Befestigungselemente 280 können hakenförmig ausgebildet sein. Vorzugsweise sind die Befestigungselemente 280 an dem äußeren Rand 276 des Funktionselementkörpers 266 angeordnet. Die Befestigungselemente 280 können in Eingriff mit den Aufnahmeelementen 62 gebracht werden, um das Funktionselement 264 zu befestigen. Je ein Befestigungselement 280 kann dabei in Eingriff mit einem entsprechenden Aufnahmeelement 62 gebracht werden.

Zum Transfer von Nährbodenträger in den Isolator 12 können die Nährbodenträger zunächst in den Nährbodenträgerhaltern 268 in dem Beta-Behälter 238 bereitgestellt werden. Dann kann der Beta-Port 44 mit dem Alpha-Port 26 gekoppelt und die Tür 32 gemeinsam mit dem Abdeckelement 50 geöffnet werden. Dann kann die Handhabungseinrichtung 216 die Nährbodenträger einzeln greifen und aus dem Beta-Behälter 238 in den Isolator 12, vorzugsweise an eine definierte Position innerhalb des Isolators 12, transferieren.

Zum Transfer von Nährbodenträgern aus dem Isolator 12 kann ebenfalls das Transfersystem 224 verwendet werden. Wenn der Beta-Port 44 mit dem Alpha-Port 26 gekoppelt und die Tür 32 mit dem Abdeckelement 50 geöffnet ist, kann die Handhabungseinrichtung 216 die Nährbodenträger einzeln aus dem Isolator 12 in den Beta-Behälter 238 transferieren und in jeweils einem der Nährbodenträgerhalter 268 anordnen. Der Beta-Port 44 und der Alpha-Port 26 können dann verschlossen und entkoppelt werden.

Des Weiteren weist die vorliegende Offenbarung Ausführungsformen gemäß den folgenden Klauseln auf:
Klausel 1: Funktionselement für einen Beta-Behälter eines Transfersystems, wobei das Funktionselement an und/oder in einer Beta-Portöffnung eines Beta-Ports des Beta-Behälters anordenbar ist, wobei das Funktionselement eine Funktionselementöffnung aufweist, wobei die Funktionselementöffnung kleiner als die Beta-Portöffnung ist.
Klausel 2: Funktionselement nach Klausel 1, wobei die Funktionselementöffnung kreisförmig ausgebildet ist.
Klausel 3: Funktionselement nach Klausel 1 oder 2, wobei die Funktionselementöffnung konzentrisch zu der Beta-Portöffnung anordenbar ist.
Klausel 4: Funktionselement nach einer der Klauseln 1 bis 3, wobei das Funktionselement ringförmig oder trichterförmig ausgebildet ist.
Klausel 5: Funktionselement für einen Beta-Behälter eines Transfersystems, wobei das Funktionselement an und/oder in einer Beta-Portöffnung eines Beta-Ports des Beta-Behälters anordenbar ist, wobei das Funktionselement einen oder mehrere Nährbodenträgerhalter aufweist, wobei jeder Nährbodenträgerhalter dazu eingerichtet ist, einen Nährbodenträger zu halten.
Klausel 6: Funktionselement nach einer der Klauseln 1 bis 5, wobei das Funktionselement als ein Einsatzelement ausgebildet ist, das in den Beta-Behälters, insbesondere in die Beta-Portöffnung, einsetzbar ist.
Klausel 7: Funktionselement nach einer der Klauseln 1 bis 6, wobei das Funktionselement einen Funktionselementkörper aufweist, wobei der Funktionselementkörper die Funktionselementöffnung umgibt oder an dem Funktionselementkörper die ein oder mehreren Nährbodenträgerhalter angeordnet sind.
Klausel 8: Funktionselement nach Klausel 7, wobei der Funktionselementkörper einen äußeren Rand aufweist, wobei der äußere Rand elastisch, insbesondere lamellenartig, ausgebildet ist.
Klausel 9: Funktionselement nach Klausel 7 oder 8, wobei der Funktionselementkörper einen äußeren Rand aufweist, wobei der äußere Rand komplementär zu der Beta-Portöffnung ausgebildet ist.
Klausel 10: Funktionselement nach einer der Klauseln 1 bis 9, wobei das Funktionselement ein oder mehrere Befestigungselemente aufweist, mittels denen das Funktionselement an dem Beta-Port, insbesondere an einem Beta-Portgrundkörper des Beta-Ports, befestigbar ist.
Klausel 11: Beta-Behälter für ein Transfersystem, wobei der Beta-Behälter ein Innenvolumen, eine Außenwand, die das Innenvolumen umschließt, einen Beta-Port, der mit einem Alpha-Port des Transfersystems koppelbar ist, und das Funktionselement nach einer der Klauseln 1 bis 10 aufweist.
Klausel 12: Beta-Behälter nach Klausel 11, wobei der Beta-Port das Funktionselement aufweist, insbesondere wobei ein Beta-Portgrundkörper des Beta-Ports und das Funktionselement einstückig ausgebildet sind.
Klausel 13: Beta-Behälter nach Klausel 11 oder 12, wobei der Beta-Port ein Abdeckelement zum Abdecken der Beta-Portöffnung aufweist, wobei das Funktionselement zwischen dem Abdeckelement und dem Innenvolumen angeordnet ist.
Klausel 14: Beta-Behälter nach einer der Klauseln 11 bis 13, wobei der Beta-Port ein Dichtungselement zum Abdichten der Kopplung zwischen dem Alpha-Port und dem Beta-Port aufweist.
Klausel 15: Transfersystem, wobei das Transfersystem einen Alpha-Port und den Beta-Behälter nach einer der Klauseln 11 bis 14 aufweist.
Klausel 16: Barrieresystem, wobei das Barrieresystem das Transfersystem nach Klausel 15 aufweist, insbesondere wobei das Barrieresystem ein Isolatorsystem ist.
Klausel 17: Barrieresystem nach Klausel 16, wobei das Barrieresystem einen Isolator aufweist, wobei der Isolator einen Innenraum und eine Isolatorwand aufweist, die den Innenraum umschließt, wobei der Alpha-Port an der Isolatorwand angeordnet ist, wobei der Beta-Port von einer äußeren Umgebung des Isolators an den Alpha-Port koppelbar ist.
Klausel 18: Barrieresystem nach Klausel 17, wobei der Alpha-Port an einer vertikalen Wandfläche der Isolatorwand angeordnet ist.
Klausel 19: Barrieresystem nach einer der Klauseln 16 bis 18, wobei das Barrieresystem des Weiteren eine Zuführeinrichtung in dem Innenraum des Isolators aufweist, wobei die Zuführeinrichtung von Innen an dem Alpha-Port anordenbar ist.
Klausel 20: Barrieresystem nach Klausel 19, wobei die Zuführeinrichtung ein Rohr aufweist, wobei ein erstes, offenes Ende des Rohrs an dem Alpha-Port anordenbar ist, insbesondere wobei das Rohr zylindrisch ausgebildet ist.
Klausel 21: Barrieresystem nach Klausel 19 oder 20, wobei das Rohr der Zuführeinrichtung an dem Alpha-Port derart anordenbar ist, dass es sich im gekoppelten Zustand durch eine Alpha-Portöffnung des Alpha-Ports hindurch bis zu der Beta-Portöffnung erstreckt, insbesondere wobei das erste Ende des Rohrs an einem Funktionselementflansch des Funktionselements anordenbar ist.
Klausel 22: Barrieresystem nach einer der Klauseln 19 bis 21, wobei das Rohr der Zuführeinrichtung an dem Alpha-Port derart anordenbar ist, dass das Rohr schräg zu einer horizontalen Richtung verläuft, insbesondere wobei das erste Ende des Rohrs eine elliptische Form aufweist.

## Patentansprüche

1. Funktionselement (264) für einen Beta-Behälter (238) eines Transfersystems (224), wobei das Funktionselement (264) an und/oder in einer Beta-Portöffnung (48) eines Beta-Ports (44) des Beta-Behälters (238) anordenbar ist, wobei das Funktionselement (264) einen oder mehrere Nährbodenträgerhalter (268) aufweist, wobei jeder Nährbodenträgerhalter (268) dazu eingerichtet ist, einen Nährbodenträger zu halten, wobei das Funktionselement (264) als ein Einsatzelement ausgebildet ist, das in den Beta-Behälters (238) einsetzbar ist, wobei das Funktionselement (264) ein oder mehrere Befestigungselemente (280) aufweist, mittels denen das Funktionselement (264) an dem Beta-Port (44) befestigbar ist.

2. Funktionselement (264) nach Anspruch 1, wobei das Einsatzelement in die Beta-Portöffnung (48) einsetzbar ist.

3. Funktionselement (264) nach Anspruch 1 oder 2, wobei das Funktionselement (264) einen Funktionselementkörper (266) aufweist, wobei die ein oder mehreren Nährbodenträgerhalter (268) an dem Funktionselementkörper (266) angeordnet sind.

4. Funktionselement (264) nach Anspruch 3, wobei der Funktionselementkörper (266) einen äußeren Rand (276) aufweist, wobei der äußere Rand (276) elastisch, insbesondere lamellenartig, ausgebildet ist.

5. Funktionselement (264) nach Anspruch 3 oder 4, wobei der Funktionselementkörper (266) einen äußeren Rand (276) aufweist, wobei der äußere Rand (276) komplementär zu der Beta-Portöffnung (48) ausgebildet ist.

6. Funktionselement (264) nach einem der Ansprüche 1 bis 5, wobei das Funktionselement (264) mittels den ein oder mehreren Befestigungselementen (280) an einem Beta-Portgrundkörper (46) des Beta-Ports (44) befestigbar ist.

7. Beta-Behälter (238) für ein Transfersystem (224), wobei der Beta-Behälter (238) ein Innenvolumen (42), eine Außenwand (40), die das Innenvolumen (42) umschließt, einen Beta-Port (44), der mit einem Alpha-Port (26) des Transfersystems (224) koppelbar ist, und das Funktionselement nach (264) einem der Ansprüche 1 bis 6 aufweist.

8. Beta-Behälter (238) nach Anspruch 7, wobei der Beta-Port (44) ein Abdeckelement (50) zum Abdecken der Beta-Portöffnung (48) aufweist, wobei das Funktionselement (64, 164, 264) zwischen dem Abdeckelement (50) und dem Innenvolumen (42) angeordnet ist.

9. Beta-Behälter (38, 138, 238) nach Anspruch 7 oder 8, wobei der Beta-Port (44) ein Dichtungselement (60) zum Abdichten der Kopplung zwischen dem Alpha-Port (26) und dem Beta-Port (44) aufweist.

10. Transfersystem (224), wobei das Transfersystem (224) einen Alpha-Port (26) und den Beta-Behälter (238) nach einem der Ansprüche 7 bis 9 aufweist.

11. Barrieresystem, wobei das Barrieresystem das Transfersystem (224) nach Anspruch 10 aufweist, insbesondere wobei das Barrieresystem ein Isolatorsystem ist.

12. Barrieresystem nach Anspruch 11, wobei das Barrieresystem einen Isolator (12) aufweist, wobei der Isolator (12) einen Innenraum (14) und eine Isolatorwand aufweist, die den Innenraum umschließt, wobei der Alpha-Port (26) an der Isolatorwand angeordnet ist, wobei der Beta-Port (44) von einer äußeren Umgebung (15) des Isolators (12) an den Alpha-Port (26) koppelbar ist.

13. Barrieresystem nach Anspruch 12, wobei der Alpha-Port (26) an einer vertikalen Wandfläche der Isolatorwand angeordnet ist.
